# EUROPEAN PATENT SPECIFICATION

(11) **EP 3 285 925 B1**
(45) Date of publication and mention of the grant of the patent: **03.07.2019**
(21) Application number: 16718335.9
(22) Date of filing: 22.04.2016
(51) Int. Cl.: C12Q 1/686

(54) **DIGITAL PCR SYSTEMS AND METHODS USING DIGITAL MICROFLUIDICS**
DIGITALE PCR-SYSTEME UND VERFAHREN MIT DIGITALER MIKROFLUIDIK
SYSTÈMES ET PROCÉDÉS DE PCR NUMÉRIQUES UTILISANT LA MICROFLUIDIQUE NUMÉRIQUE

(30) Priority: 24.04.2015 US 201562152581 P
(43) Date of publication of application: 28.02.2018
(73) Proprietor: Roche Diagnostics GmbH, 68305 Mannheim (DE); F. Hoffmann-La Roche AG, 4070 Basel (CH)
(72) Inventor: KWANG, Stanford, Pleasanton, California 94588 (US)
(74) Representative: Schwarz, Ralf
(86) International application number: PCT/EP2016/059001
(87) International publication number: WO 2016/170109

(56) References cited:
- EP-A1- 2 570 188
- WO-A1-2012/031050
- US-A1- 2004 055 891
- US-A1- 2013 017 544
- A. DIDELOT ET AL: "Multiplex Picoliter-Droplet Digital PCR for Quantitative Assessment of DNA Integrity in Clinical Samples", CLINICAL CHEMISTRY, vol. 59, no. 5, 12 February 2013 (2013-02-12), pages 815-823, XP55197965, ISSN: 0009-9147, DOI: 10.1373/clinchem.2012.193409

## Description

### FIELD OF THE INVENTION

The present invention relates to the field of nucleic acid amplification, and more particularly, to systems and methods for digital polymerase chain reaction using digital microfluidics.

### BACKGROUND OF THE INVENTION

Digital polymerase chain reaction (dPCR) is a refinement of conventional PCR and can be used to directly quantify and clonally amplify nucleic acids, e.g., DNA, cDNA or RNA. Conventional PCR is generally used for measuring nucleic acid amounts and is carried out by a single reaction per sample. Utilizing dPCR methodology, a single reaction is also carried out on a sample, however the sample is separated into a large number of partitions and the reaction is carried out in each partition individually. This separation allows for a more reliable collection and sensitive measurement of nucleic acid amounts.

In dPCR, a sample is partitioned so that individual nucleic acid molecules within the sample are localized and concentrated within many separate regions. The capture or isolation of individual nucleic acid molecules can be performed in micro well plates, capillaries, the dispersed phase of an emulsion, and arrays of miniaturized chambers, as well as on nucleic acid binding surfaces. The partitioning of the sample allows one to estimate the number of different molecules by assuming that the molecule population follows the Poisson distribution. As a result, each partitioned sample will contain "0" or "1" molecules, or a negative or positive reaction, respectively. After PCR amplification, nucleic acids can be quantified by counting the regions that contain PCR end-product, positive reactions. In conventional PCR, the number of PCR amplification cycles is proportional to the starting copy number. dPCR, however, is not dependent on the number of amplification cycles to determine the initial sample amount, eliminating the reliance on uncertain exponential data to quantify target nucleic acids and therefore provides absolute quantification.

The existing dPCR systems and methods have fragmented workflows requiring user intervention. Thus, there is a need in the art for a more efficient and reliable system and method to perform dPCR reactions and analysis.

### SUMMARY OF THE INVENTION

Systems and methods are described for performing digital PCR using digital microfluidics configured for precise movement of picoliter to nanoliter sized partitions which can be used for partition generation, movement through a temperature gradient for PCR and nucleic acid melting, and signal detection, all within a single consumable device. Additionally, the user can perform quantitative multiplexing using high resolution melting/melting curves techniques [1, 2]. The prior art describes microfluidic devices for carrying out amplification, melting analysis and droplet movements (US20130017544 A1, WO2012031050 A1, EP2570188 A1).

In one embodiment, a digital PCR system is provided, including a microfluidic device, having a sample loading zone comprising at least one well for receiving a fluid sample; a serial dilution zone comprising a first reagent reservoir for diluting the sample; a PCR set up zone comprising a second reagent reservoir; a partition generation zone configured to generate a plurality of partitions of the sample; a PCR zone comprising at least one thermal region comprising a first temperature region and a second temperature region defining a thermal protocol for PCR amplification; and a melt curve zone comprising a thermal gradient configured to generate a melting profile of a PCR amplification product.

In a specific embodiment, a device is provided including an upper and lower substrate and a lateral plane positioned between the upper and lower substrate, the lower substrate comprising an electrode array configured to move a partition along the lateral plane, wherein the lateral plane includes a plurality of zones comprising, from a proximate to a distal end, (a) a preparation zone comprising (i) a sample loading zone, (ii) a water reservoir and one or more reagent reservoirs each in communication with the sample loading zone; and (iii) a partition generation zone; (b) an amplification zone in thermal communication with one or more heating elements configured to subject the amplification zone to a thermal protocol for PCR amplification, and (c) a melt curve zone in thermal communication with one or more additional heating elements configured to subject the melt curve zone to a thermal gradient to generate a melting profile of an amplification product.

In addition, also provided is a method of performing digital PCR on an electrowetting-based microfluidic device, the method comprising the steps in the following order: (a) adding a partition comprising a sample to a sample loading zone positioned on the device, (b) diluting the partition with a volume of water; (c) mixing the partition with a PCR reagent mixture; (d) partitioning the partition into a plurality of partitions; (e) subjecting the plurality of partitions to a thermal protocol to generate one or more amplicon-containing partitions; and (f) subjecting the one or more amplicon-containing partitions to a thermal gradient and thereby generate a melting profile for each of the one or more amplicon-containing partitions. In a specific embodiment of the method, a first set of partitions are subjected to steps (a)-(f); and one or more additional sets of partitions are subjected to steps (a)-(f), wherein the volume of sample in the partitions in the one or more additional sets is smaller than the volume of sample in the first set, wherein the method is repeated until an optimal Poisson distribution is achieved. In addition, the method can include subjecting a first set of partitions to steps (a)-(f) and one or more additional sets of partitions are subjected to steps (a)-(f), wherein the sample in the one or more additional sets of partitions is serially diluted relative to the sample in the first set of partitions. In this regard, one or more subsequent sets of partitions can be subjected to steps (a)-(f), wherein the sample in the one or more subsequent sets of partitions is serially diluted relative to the sample in the first and one or more additional sets of partitions. Moreover, the disclosure provides a method of performing a multiplexed digital PCR analysis on an electrowetting-based microfluidic device, the method comprising the steps in the following order: (a) adding a partition comprising a sample to a sample loading zone positioned on the device, wherein the sample comprises a plurality target sequences, (b) diluting the partition with a volume of water; (c) mixing the partition with a PCR reagent mixture; (d) partitioning the partition into a plurality of partitions; (e) subjecting the plurality of partitions to a thermal protocol to generate one or more amplicon-containing partitions; (f) subjecting the one or more amplicon-containing partitions to a thermal gradient and thereby generate a melting profile for each of the one or more amplicon-containing partitions; and (g) detecting the presence and/or absence of each of the target sequences in the plurality of target sequences based on the melting profile for each of the one or more amplicons. In this embodiment, a first set of partitions are subjected to steps (a)-(g); and one or more additional sets of partitions are subjected to steps (a)-(g), wherein the volume of sample in the partitions in the one or more additional sets is smaller than the volume of sample in the first set, wherein the method is repeated until an optimal Poisson distribution is achieved. Moreover, a first set of partitions can be subjected to steps (a)-(g) and one or more additional sets of partitions are subjected to steps (a)-(g), wherein the sample in the one or more additional sets of partitions is serially diluted relative to the sample in the first set of partitions. Still further, one or more subsequent sets of partitions are subjected to steps (a)-(g), wherein the sample in the one or more subsequent sets of partitions is serially diluted relative to the sample in the first and one or more additional sets of partitions.

Finally, in the methods described herein, following step (e), each of the plurality of partitions comprises zero or one target sequence.

The details of one or more embodiments of the present subject matter are set forth in the accompanying drawings and the description below. Other features, objects, and advantages will be apparent from the drawings and detailed description, and from the claims.

### BRIEF DESCRIPTION OF THE FIGURES

Fig. 1 illustrates certain subcomponents of a microfluidic device including upper and lower substrates and a lateral plane positioned therebetween, wherein the lateral plane includes a plurality of zones described herein.

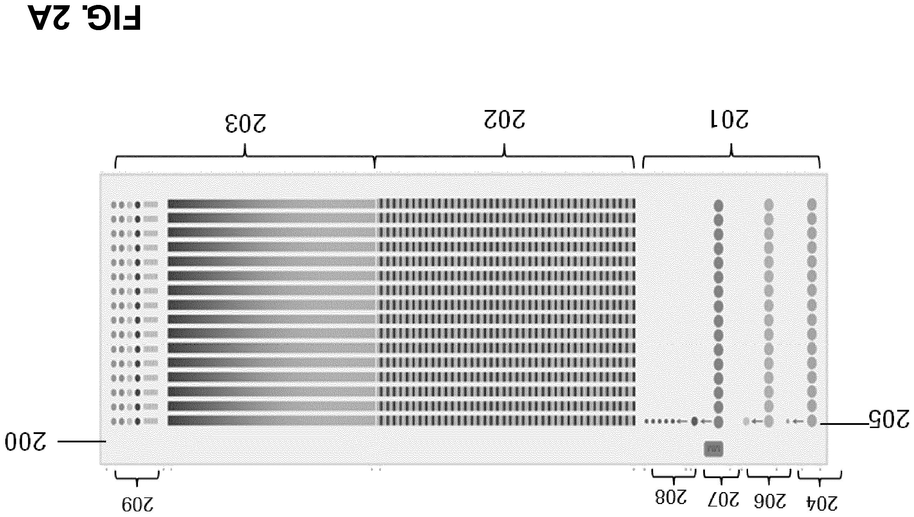

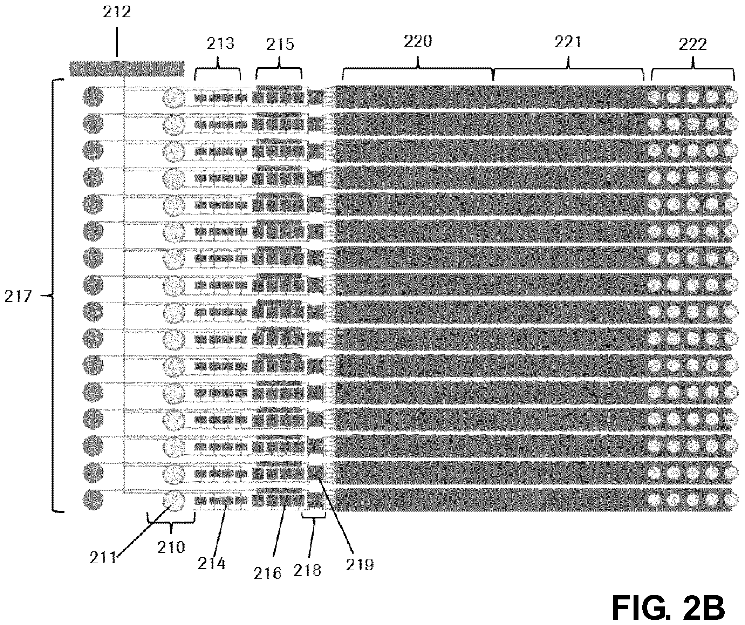

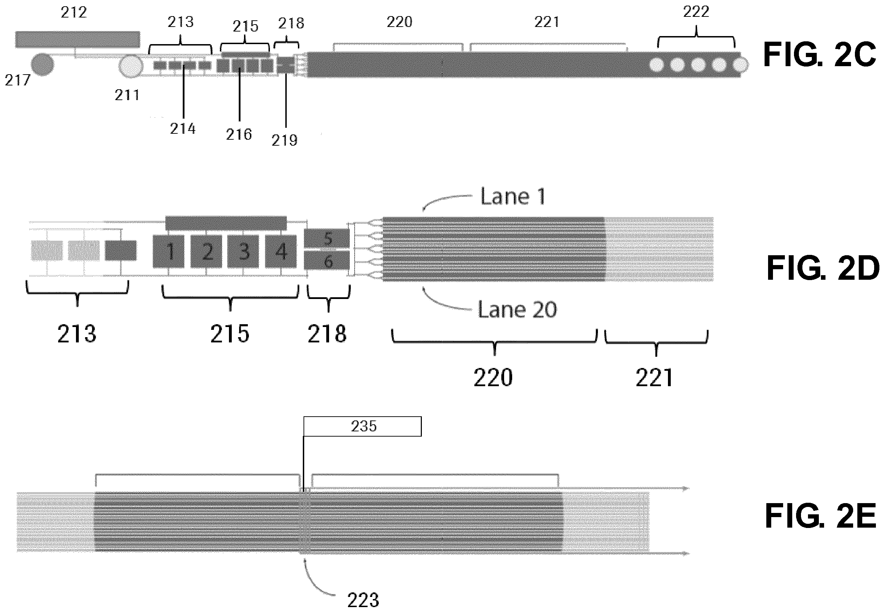

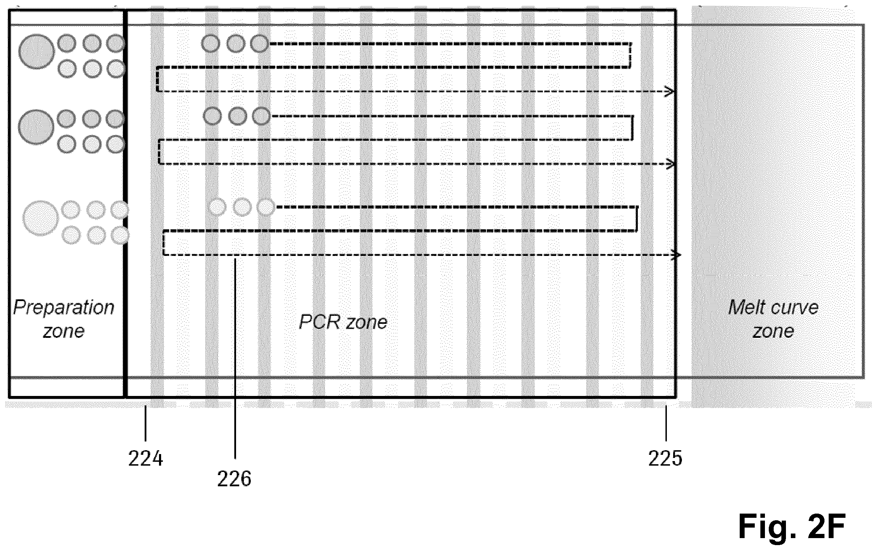

Figs. 2A-2F illustrate additional subcomponents of a microfluidic device. Fig. 2A shows a single consumable device (200) comprising, from a proximate to a distal end, a preparation zone (201), an amplification zone (202), and a melt curve zone (203). Figs. 2B-2C illustrate an alternative embodiment of the preparation zone of device 200, and Fig. 2D shows an expanded view of the sample dilution staging zone, PCR reagent staging zone, partition generation staging zone, and the amplification and melt curve zones. Fig. 2E illustrates an embodiment of the device that includes a partition sorting zone. And Fig. 2F shows a specific configuration of the amplification zone.
Fig. 2G shows a system configured to use device 200.
Fig. 3A illustrates how device 200 is used for retesting of the same sample and Fig. 3B illustrates the repetitive dilution of a portion of sample until a desired degree of precision is achieved.
Fig. 4 illustrates how melting curve and HRM analysis in real-time PCR is not equivalent to that performed by digital PCR.
Figs. 5A-5C illustrate how the device and method described herein can be used for multiplexed dPCR measurements.
Figs. 6A-6D illustrate four scenarios describing quantitative multiplexing with and without dynamic partitioning with samples high in target concentration and low in target concentration.
Fig. 7 illustrates the impact of low positive partitions from Bio-Rad's QX200 analysis software.
Fig. 8 shows the use of HRM or melt curve data to better discriminate results obtained with low positive partitions.
Fig. 9 illustrates sample turnaround time using digital microfluidic technology.
Fig. 10 shows a chart illustrating sample throughput per hour.
Figs. 11A-11C show the time required to process successive dilutions in serial order.

### DETAILED DESCRIPTION OF THE INVENTION

For the purposes of the present disclosure, the term "communicate" is used to indicate a structure, functional, mechanical, optical, thermal, or fluidic relation, or any combination thereof, between two or more components or elements. The fact that one component is said to communicate with a second component is not intended to exclude the possibility that additional components may be present between and/or operatively associated or engaged with the first and the second components.

In addition, for the purposes of this disclosure, it will be understood that when a liquid in any form (e.g., a partition, droplet or a continuous body, whether moving or stationary) is described as being "on," "at," or "over" a surface, electrode, array or device, such liquid could be either in direct contact with a surface, electrode, array, or device, or component thereof, or it could be in contact with one or more layers or films interposed between the liquid and the surface, electrode, array, or device, or component thereof.

For the purposes of this disclosure, a partition is a separated portion of a bulk volume. The partition may be a sample partition generated from a sample, such as a prepared sample, that forms the bulk volume. Partitions may be substantially uniform in size or may have distinct sizes (e.g., sets of partitions of two or more discrete, uniform sizes). Exemplary partitions are droplets. Partitions may also vary continuously in size with a predetermined size distribution or with a random size distribution. A droplet is an example of a partition and as used herein, a droplet is a small volume of liquid, typically with a spherical shape, encapsulated by an immiscible fluid, such as a continuous phase of an emulsion. The volume of a droplet, and/or the average volume of droplets in an emulsion, may, for example, be less than about one microliter, less than about one nanoliter, or less than about one picoliter. A droplet (or droplets of an emulsion) may have a diameter (or an average diameter) of less than about 1000, 100, or 10 micrometers, or of about 1000 to 10 micrometers, among others. A droplet may be spherical or nonspherical. A droplet may be a simple droplet or a compound droplet, that is, a droplet in which at least one droplet encapsulates at least one other droplet.

As used herein, the term "reagent" describes any agent or a mixture of two or more agents useful for reacting with, diluting, solvating, suspending, emulsifying, encapsulating, interacting with, or adding to a sample. A reagent can be living such as a cell or non-living. Reagents for a nucleic acid amplification reaction include, but not limited to, buffer, polymerase, primers, template nucleic acid, nucleotides, labels, dyes, nucleases, etc.

Digital microfluidics use electrowetting or dielectrophoresis to manipulate discrete partitions, e.g., droplets, of liquid by leveraging a combination of surface tension and electric fields. First implemented in 1987 at Cytonix, digital microfluidics enables the use of electric fields to move partitions across a surface. Briefly, when a liquid partition is placed on a hydrophobic surface the partition forms a droplet on the surface. When an electric field is applied, the surface becomes hydrophilic resulting in the liquid partition adhering to the surface. By varying the surrounding surfaces of a partition on a hydrophobic surface, the partition will migrate to the electrified hydrophilic surface resulting in partition movement. Therefore, partitions can be moved around a surface at greater and greater speed. It has been demonstrated that nanoliter droplets can be made to migrate at 90 Hz [3]. Embodiments of the present subject matter incorporate digital microfluidic technology and apply it to digital PCR.

A melting curve (dissociation curve) charts the change in fluorescence observed when double-stranded DNA dissociates or "melts" into single-stranded DNA as the temperature of the reaction is raised. For example, when double-stranded DNA is heated, a sudden decrease in fluorescence is detected with the melting point (Tm) is reached. Post-amplification melting-curve analysis can be used to detect primer-dimer artifacts and contamination and to ensure reaction specificity. Because the Tm of nucleic acids is affected by length, GC content, and the presence of base mismatches, among other factors, different PCR products can be distinguished by their melting characteristics. Moreover, the characterization of reaction products, e.g., primer-dimers vs. amplicons via melting curve analysis reduced the need for time-consuming gel electrophoresis. The specificity of a real-time PCR assay is determined by the primers and reaction conditions used. However, there is always the possibility that even well-designed primers may form primer-dimers or amplify a nonspecific product. There is also the possibility when performing qRT-PCR that the RNA sample contains genomic DNA, which may also be amplified. The specificity of the qPCR or qRT-PCR reaction can be confirmed using melting curve analysis.

High-resolution melt curve (HRM) analysis is a homogeneous, post-PCR method for identifying, e.g., SNPs, novel mutations, and methylation patterns. HRM analysis is a more sensitive approach to traditional melt curve profiling, in which double-stranded DNA is monitored for the temperature at which is dissociates into single-stranded DNA (Tm). After amplification, the instrument slowly increases the temperature while simultaneously monitoring fluorescence. The fluorescence level slowly decreases until the temperature approaches the product Tm and very close to the Tm, a dramatic decrease in fluorescence is observed as the sample transitions from double stranded to single stranded DNA. A specific DNA sequence has a characteristic profile. Mutations are detected as either a shift in Tm or as a change in shape of the melting curve. In contrast to traditional melt curve analysis, HRM can provide single-nucleotide discrimination between amplicons.

The device and methods described herein are configured to generate a melting profile for the products generated in the amplification zone. As used herein, a "melting profile" includes a traditional melt curve analysis as well as HRM analysis.

One embodiment of the device described herein is a microfluidic device comprising an upper and lower substrate and a lateral plane positioned between the upper and lower substrates. The lower substrate includes an electrode array configured to move a partition along the lateral plane. Additionally or alternatively, the electrode can be positioned in the upper substrate (the device is described herein as having the electrode positioned on the lower substrate but it will be understood by those skilled in the art that the alternative configuration in which the electrode is positioned on the upper substrate is a suitable alternative). The lateral plane of the device includes a plurality of zones comprising, from a proximate to a distal end,
(a) a preparation zone comprising (i) a sample loading zone, (ii) a water reservoir and one or more reagent reservoirs each in communication with the sample loading zone; and (iii) a partition generation zone;
(b) an amplification zone in thermal communication with one or more heating elements configured to subject the amplification zone to a thermal protocol for polymerase chain reaction (PCR) amplification, and
(c) a melt curve zone in thermal communication with one or more additional heating elements configured to subject the melt curve zone to a thermal gradient to generate a melting profile of an amplification product.

This embodiment of a microfluidic device and the method of using it are described in more detail below.

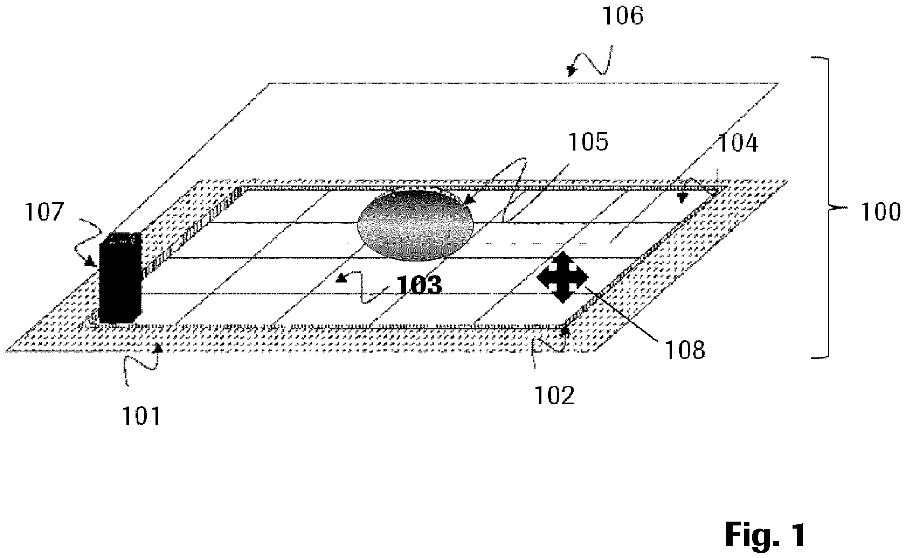

Fig. 1 illustrates the upper and lower substrates and the lateral plane positioned there between. Device, 100, comprises a lower substrate, 101, with thin film electronics, 102, positioned on the lower substrate. The electronics are arranged to drive one or more array element electrodes, e.g., 103. A plurality of array element electrodes, 103, is arranged in an electrode array, 104, having MxN elements wherein M and N are integers. In a specific embodiment, M and N are each equal to or greater than 2. A liquid partition, e.g., droplet 105, is enclosed between the lower and upper substrates (101 and 106, respectively) (multiple partitions can be positioned between the lower and upper substrates). Spacer, 107, is disposed between the lower and upper substrates, positioned in the device to generate a suitable gap between the two substrates and a non-ionic fluid (not shown), e.g., oil, fills the volume not occupied by the partition. By appropriate design and operation of the thin film electronics, different voltages are applied to different electrodes in the array, thereby controlling the hydrophobicity of the surface and facilitating partition movement in the lateral plane (108) between the upper and lower substrates. In this regard, a fluidic network in the lateral plane is generated in the device by varying the voltage applied to one or more segments of the electrode array to migrate a partition from one zone or region of the device to another. Additional details of a suitable electrowetting-based digital microfluidic device are described in U.S. Application Publication No. 2013/0062205.

Figs. 2A-2F illustrate additional components of the device shown in Fig. 1. Fig. 2A shows a single consumable device (200) comprising, from a proximate to a distal end, a preparation zone (201), an amplification zone (202), and a melt curve zone (203). The elements of each zone are described in more detail below.

The preparation zone includes a sample loading zone (204), including a plurality of sample loading regions (e.g., 205) each configured to accommodate a partition, e.g., a droplet including an emulsified volume of sample. In a specific embodiment, each sample loading region is operatively connected to at least one lane or path on the device along which the partition migrates from one zone to the next. As described above in reference to Fig. 1, each lane or path is defined by the appropriate application of voltage to one or more elements of an electrode array positioned in the device. Therefore, the lane or path is linear or non-linear. Each sample loading region includes a sample inlet (not shown), configured to accept a syringe, pipette or a PCR tube containing sample. The device shown in Figs. 2A-2F comprises a plurality of sample loading regions, e.g., sixteen discrete regions, which can be loaded with a multi-channel pipette. However, the skilled artisan will readily appreciate that this configuration can be adjusted based on the user's needs. The preparation zone also includes a water reservoir or a series of water reservoirs (206) and one or more reagent reservoirs (207) each in communication with one or more sample loading regions. In a specific embodiment, sample is added to the sample loading zone, which is operatively connected to the water and reagent reservoirs, such that the sample partition is diluted and mixed with reagent by migrating the partition along the lateral plane of the device from one region of the preparation zone to another via the controlled application of voltage to the electrode array in one or more regions of the preparation zone. Once the initial partition of sample is diluted and mixed with reagents, the partition is subsequently moved along the lateral plane of the device to a partition generation zone (208) positioned in the preparation zone. The partition generation zone is configured to further divide a partition and further partitioning is accomplished by the suitable application of voltage to the electrode array in the partition generation zone.

The partition is then migrated from the preparation zone into the amplification zone (202) where it is subjected to a thermal protocol for amplification. The amplification zone is in thermal communication with one or more heating elements (not shown) that are configured to increase the temperature in the amplification zone from a proximate to a distal end. For example, the amplification zone is in thermal communication with at least two heating elements, the first heating element in the amplification zone configured to heat the a denaturation region of the amplification zone (not shown) to a temperature at which the DNA is denatured, e.g., 95°C, and the second heating element is configured to heat an annealing region of the amplification zone (not shown) to a temperature at which the DNA is annealed, e.g., the annealing temperature is about 5°C below the Tm of the primers. The optimal annealing temperature (Ta Opt) for any given primer pair on a particular target can be calculated as follows: Ta Opt = 0.3 x(Tm of primer) + 0.7 x(Tm of product) - 25; where Tm of primer is the melting temperature of the less stable primer-template pair, and Tm of product is the melting temperature of the PCR product. In a specific embodiment, the partition is migrated along a path in the amplification zone which is linear or non-linear. The path is optionally circuitous such that the partition migrates back and forth across the zone between the denaturation and annealing regions until the desired number of cycles is reached. The amplification zone can be in optical communication with a detection system (not shown) configured to detect an optical signal emitted from a partition in the amplification zone. Once amplification is completed in the amplification zone, each partition migrates to the melt curve zone (203) which is configured to subject the partition to a temperature gradient to generate a melting profile. Like the amplification zone, the melt curve zone is in thermal communication with one or more additional heating elements configured to subject the partition migrating through the melt curve zone to a temperature gradient. The melt curve zone is in optical communication with a detection system (not shown) configured to detect an optical signal emitted from a partition in the melt curve zone. Optionally, partitions are collected into various isolation reservoirs (209) in order to aid in downstream workflows, if needed, such as sequencing. This approach of complete integration of workflow can also be used for isothermal amplification techniques as well with the amplification zone calibrated to one temperature.

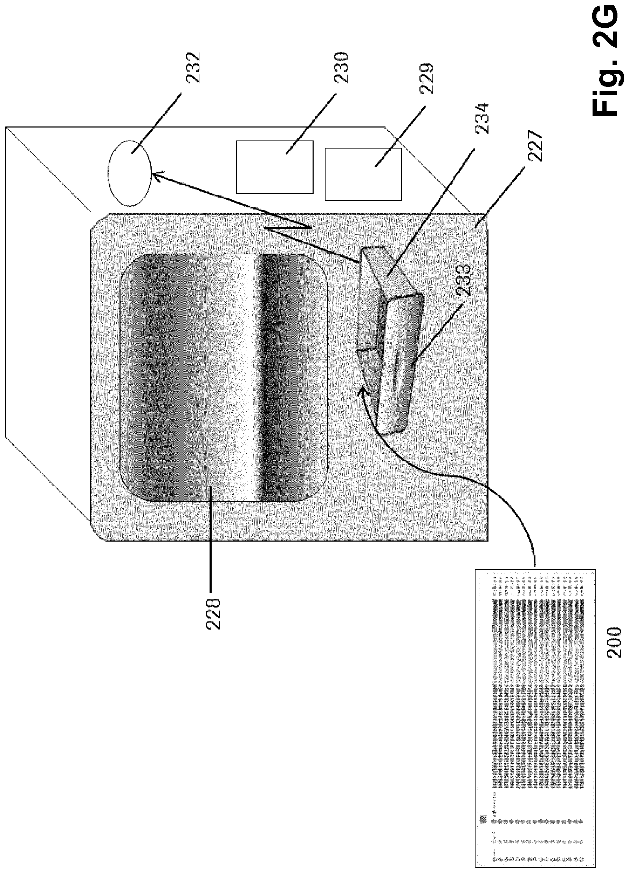

As described above in reference to the amplification and melt curve zones, the device is thermally associated with one or more heating elements controlled by an operating system that operates the device and one or more components of an associated system in which the device is operated (see, e.g., Fig. 2G). The operating system includes a processor, e.g., a computer, configured to actuate the one or more heating elements according to a desired protocol.

A detailed view of an alternative embodiment of the preparation zone of device, 200, is shown in Figs. 2B-2C. Sample is introduced via sample loading zone (210) comprising a plurality of sample loading regions (e.g., 211), each configured to accommodate a partition, e.g., a droplet including an emulsified volume of sample. The sample loading zone is in communication with a common water reservoir (212). The partition is migrated via electrowetting from the sample loading region to the sample dilution staging zone (213, comprising a plurality of dilution chambers, e.g., 214). Once diluted, the partition is migrated to the PCR reagent staging zone (215, comprising a plurality of staging chambers, e.g., 216). The PCR reagent staging zone is in communication with one or more PCR reagent reservoirs (217), such that the partition is mixed via electrowetting with a suitable volume and concentration of PCR reagents. The partition is migrated from the PCR reagent staging zone to the partition generation staging area (218, including a plurality of partition generation staging chambers, e.g., 219), where it is further partitioned before migrating to the amplification zone (220) where it is subjected to a thermal protocol for amplification. Once amplification is done, each partition is passed through the melt curve zone (221) which is configured to subject a partition to a temperature gradient to generate a melting profile. Finally, partitions are collected into various isolation reservoirs (222) in order to aid in downstream workflows, if needed, such as sequencing. Fig. 2C includes an expanded view of one lane of a device (200).

Fig. 2D provides an expanded view of the sample dilution staging zone (213), PCR reagent staging zone (215), partition generation staging zone (218), and the amplification zone (220) and melt curve zone (221). As shown in Fig. 2D, a single partition is further partitioned into a plurality of partitions, and each migrates into a plurality of lanes, e.g., up to twenty lanes, in the amplification zone and melt curve zones. Moreover, in order to account for the fact that many partitions may be negative after amplification, the device can be adapted to include a partition sorting zone (223, shown in Fig. 2E) that will facilitate removal of negative partitions before migrating to the melt curve zone. Negative partitions can be migrated to a waste chamber (235) and position/negative partitions can be analyzed at the distal end of the amplification zone, e.g., via optical detection of a detectable signal from each partition. Therefore, the amplification zone is optionally in optical communication with a detection system configured to detect an optical signal from each partition, e.g., via the emission of a fluorescence signal indicative of the presence of a positive partition. This feature reduces the number of partitions that migrate through the melt curve zone, thereby attenuating the speed of partition movement so that samples can be processed quickly.

A non-limiting example of a configuration of the amplification zone is shown in Fig. 2F. As shown in Fig. 2F, the device is in thermal communication with one or more heating and cooling elements (not shown) that are configured to increase the temperature from a proximate end (224) to a distal end (225) of the amplification zone. The channel or path (226) in which a partition migrates can be a linear path through the amplification zone or, as shown in Fig. 2F, a circuitous path that migrates the partition from the proximate to the distal end of the amplification zone and back again until the desired number of cycles is reached.

A system configured to use device 200 is shown in Fig. 2G. The system (227) includes a user-interface (228), a computer (229) operatively connected to the system including a computer readable medium having stored thereon a computer program which, when executed by the computer, causes the system to perform an analysis using the device (200), a sample/reagent introduction and preparation chamber (230), an optical detection subsystem (232), and a removable drawer (233) adapted to receive device 200. The removable drawer comprises one or more heating and cooling elements (234) configured to thermally contact the lower substrate and/or the top substrate at the amplification and melt curve zones. Alternatively, one or more heating elements can be incorporated into the top substrate of the device and the cooling elements can be in the incorporated into the drawer in thermal communication with the lower substrate of the device (not shown). The computer program comprises a system control program, including but not limited to a partition control program configured to control the selective application of voltage to one or more elements of the electrode array in one or more zones, paths, and/or lanes of the device in order to separate (partition) a partition positioned in the selected zone, path and/or lane.

Device, 200, is configured to accommodate and manipulate partitions less than 10 nL in volume, specifically less than 5 nL, more specifically approximately 2 nL (i.e., corresponding to about 210 um pixels). In a particular embodiment, the device is configured to migrate partitions less than 1 nL (corresponding to about 105 um pixels) and approximately 20,000 partitions per device. The device throughput is approximately 10-30 partitions/mm width of the device per second, i.e., a partition speed of about 36-54 el/s.

For example, the device described herein is configured to analyze a plurality of samples partitioned into at least 1,000,000. More specifically, the device can analyze a plurality of samples partitioned into at least 100,000, e.g., 20,000-50,000 partitions. In a specific embodiment, the device can analyze a plurality of samples partitioned into 20,000 partitions. The device can analyze up to 100 samples, e.g., up to 50 samples, up to 25 samples, and more specifically, up to 16 samples. Still further, the device is configured to analyze a plurality of samples in less than 500 seconds, e.g., less than 250 seconds, less than 150 seconds, and in a specific embodiment, between 125-500 seconds, or particularly between 140-460 seconds.

In a particular embodiment, the device is adapted to analyze at least 16 samples partitioned into 20,000 partitions in less than 500 seconds.

In another embodiment, the device is adapted to analyze at least 16 samples partitioned into 100,000 partitions in less than 500 seconds. The device can also be adapted to analyze at least 16 samples partitioned into 1,000,000 partitions in less than 500 seconds.

The approximate length of the active area of the device described herein is about 11-16 cm, i.e., 5-10 cm for the amplification zone, approximately 4 cm for the melt curve zone and approximately 2 cm for the preparation zone.

The device described herein is configured to perform a digital PCR analysis by the following method:
(a) adding a partition comprising a sample to a sample loading zone positioned on the device,
(b) diluting the partition with a volume of water;
(c) mixing the partition with a PCR reagent mixture;
(d) partitioning the partition into a plurality of partitions;
(e) subjecting the plurality of partitions to a thermal protocol to generate one or more amplicon-containing partitions; and
(f) subjecting the one or more amplicon-containing partitions to a thermal gradient and thereby generate a melting profile for each of the one or more amplicon-containing partitions.

In one embodiment, a first set of partitions are subjected to steps (a)-(f); and one or more additional sets of partitions are subjected to steps (a)-(f), wherein the volume of sample in the partitions in the one or more additional sets is smaller than the volume of sample in the first set, wherein the method is repeated until an optimal Poisson distribution is achieved (described in more detail below). Moreover, the method can also include subjecting a first set of partitions to steps (a)-(f) and one or more additional sets of partitions are subjected to steps (a)-(f), wherein the sample in the one or more additional sets of partitions is serially diluted relative to the sample in the first set of partitions. Still further, the one or more subsequent sets of partitions can be subjected to steps (a)-(f), wherein the sample in the one or more subsequent sets of partitions is serially diluted relative to the sample in the first and one or more additional sets of partitions.

In addition, the device described herein is configured to be used in a method of performing a multiplexed digital PCR analysis, wherein the method includes the steps in the following order:
(a) adding a partition comprising a sample to a sample loading zone positioned on the device, wherein the sample comprises a plurality target sequences,
(b) diluting the partition with a volume of water;
(c) mixing the partition with a PCR reagent mixture,;
(d) partitioning the partition into a plurality of partitions;
(e) subjecting the plurality of partitions to a thermal protocol to generate one or more amplicon-containing partitions;
(f) subjecting the one or more amplicon-containing partitions to a thermal gradient and thereby generate a melting profile for each of the one or more amplicon-containing partitions; and
(g) detecting the presence and/or absence of each of the target sequences in the plurality of target sequences based on the melting profile for each of the one or more amplicons.

In this method, a first set of partitions can be subjected to steps (a)-(g); and one or more additional sets of partitions are subjected to steps (a)-(g), wherein the volume of sample in the partitions in the one or more additional sets is smaller than the volume of sample in the first set, wherein the method is repeated until an optimal Poisson distribution is achieved. Moreover, a first set of partitions can be subjected to steps (a)-(g) and one or more additional sets of partitions are subjected to steps (a)-(g), wherein the sample in the one or more additional sets of partitions is serially diluted relative to the sample in the first set of partitions. Still further, the one or more subsequent sets of partitions can be subjected to steps (a)-(g), wherein the sample in the one or more subsequent sets of partitions is serially diluted relative to the sample in the first and one or more additional sets of partitions. Finally, in the methods described herein, following step (e), each of the plurality of partitions can include zero or one target sequences.

The device described herein can be configured to enable the entire workflow to flow seamlessly allowing for a feedback loop that can make dynamic changes to future partitions of the same sample based upon the results of analyzed partitions. The melting profile enables quantitative multiplexing as well as aid in categorizing partitions that may be difficult to discriminate as positive or negative. This consumable can be analyzed in real-time through optical imaging to track each partition as it flows through the consumable device and capture data such as signal intensity from PCR amplification and melting profile analysis. It is also possible that this device uses other means for real-time tracking and capture data.

The skilled artisan will appreciate that the relative size and shape of the device can vary based upon user requirements. Regardless of the dimensions, the device described herein is configured to enable the following on-board processes: (i) sample concentration adjustment as needed through on-board dilution; (2) partition preparation for PCR and melt curve analysis; (3) partition generation; (4) partition PCR amplification; and (5) melt curve analysis. The device configuration allows for a seamless workflow and feedback loop that can make dynamic changes to subsequent partitions of the same sample based upon the results of analyzed partitions. The melting profile facilitates quantitative multiplexing and it can also be used to identify and discriminate positive vs. negative partitions. The device can be analyzed in real-time, e.g., through optical imaging to track each partition as it flows through the device, capturing data such as signal intensity from PCR amplification and melting profile analysis. Today's digital PCR systems have fragmented workflows requiring user intervention. However, using the device described herein, numerous advantages are achieved. These advantages are described in more detail in the following non-limiting Examples.

### EXAMPLES

### Example 1. Dynamic partitioning based on sample concentration.

Dynamic range in digital PCR is based upon the number of partitions used to partition the sample[4]. Digital PCR allows the user to simply count the number of positive partitions versus the negative partitions while applying Poisson statistics to account for the random probability of more than 1 target copy per partition. Therefore, if a sample having 20,000 target copies is run through a conventional dPCR system, the user would observe approximately 12,652 positive partitions. However, some partitions have one target while others have 2, 3, 4, 5, 6, or 7 targets. Poisson statistics would predict the following distribution of 12,652 positive partitions:

**Table 1**

| Number of positive partitions | Number of copies per partitions |
|---|---|
| 7373 | 1 |
| 3636 | 2 |
| 1269 | 3 |
| 286 | 4 |
| 66 | 5 |
| 10 | 6 |
| 2 | 7 |

Using an unknown sample showing 12,652 positive partitions out of 20,000, after applying Poisson statistics, the sample will be quantified to be approximately 19,999 target copies with a standard deviation of 118 and a coefficient of variation (CV) of 0.59. If the CV is acceptable, then the user can continue to quantify the next sample. If the concentration of the unknown target is high, for example 500,000 targets, which is possible in gene expression and viral load quantitation, the same 20,000 partition system would not be able to quantify this sample with acceptable accuracy. In this scenario, given a high concentration of unknown target, Poisson statistics would predict the following positive partition distribution:

**Table 2**

| Number of positive partitions | Number of copies per partitions |
|---|---|
| 1 | 8 |
| 5 | 9 |
| 19994 | 10+ |

In these cases where the unknown sample had far more targets than can be reliably quantified by the number of partitions, the user must therefore use less of their sample, e.g., through dilution. In this scenario, a sample at 500,000 target copies per 10 microliter of sample volume could be serially diluted until an acceptable standard deviation and correlation of variance are reached:

**Table 3**

| Sample concentration (target copies/microliter) | Estimated quantity using 20,000 partitions | Standard Deviation | % CV |
|---|---|---|---|
| 500,000 | Unknown | N/A | N/A |
| 400,000 | Unknown | N/A | N/A |
| 250,000 | Unknown | N/A | N/A |
| 200,000 | 181,497 | 4,450 | 2.45% |
| 100,000 | 100,009 | 1,698 | 1.7% |
| 50,000 | 49,981 | 400 | 0.8% |

In this case, the user would need to dilute their sample by a factor of 10 in order to get from 500,000 to 50,000 with a CV of 0.89%. Unfortunately, the user does not know what the initial starting concentration is and therefore pre-quantitation is necessary to maintain an efficient workflow throughput and manage costs of repeated runs.

Ideally, one would simply increase the number of partitions so as to extend the dynamic range accommodating any unknown sample concentration. However, current digital PCR systems include a fixed number or size of partitions for each sample, e.g., using the Bio-Rad QX100/200 droplet digital PCR system, Fluidigm BioMark HD, Thermo QuantStudio 3D, Formulatrix Constellation, Raindance Raindrop systems. One approach has been to significantly increase the dynamic range using various sizes of partitions and mathematically determine the sample concentration [5]. This approach however still depends on a static number and size of partitions that cannot be customized to the sample concentration to ensure optimal concentration determination. Although microfluidic technology has enabled easy analysis of individual picoliter to nanoliter sized partitions, there are still considerable advancements needed in digital PCR to compare against the wide dynamic range of real-time PCR, which is in the range from single copy targets to 10¹⁰. To reach the dynamic range of real-time PCR, digital PCR would require samples to be partitioned to a scale too high to allow rapid testing (e.g. 10¹⁰ partitions). At best, current conventional dPCR systems can yield 10⁷ partitions per 50 microliters sample of reaction volume with considerable limitations in throughput (e.g. approximately 8 samples per 24 hours). Samples high in target copies as seen in viral load testing (e.g. Hepatitis B Virus) can reach as much as 10⁹ targets. When such samples are tested on today's digital PCR, these samples will either not be accurately quantified or some preliminary quantification step will be required, e.g., using a spectrophotometer or fluorometer. If these samples preliminarily quantify beyond the dynamic range of the digital PCR systems, the user must therefore perform some preliminary sample manipulation such as performing a serial dilution to reach the dynamic range of the system. In this scenario, the workflow is much more laborious, the throughput in samples processes is reduced, and additional error in quantitation is introduced due to a manual serial dilution step.

One solution embodied in the present disclosure is to allow for the number of partitions or the size of partitions to be flexible and dynamic based upon the concentration of the sample. In order to achieve this, the entire digital PCR workflow is integrated into a single device, from partition generation, to target amplification within the partition, and finally partition product signal detection. The approach is envisioned using digital microfluidics which provides a simple, elegant approach to create, separate, merge, and move partitions as needed. In the device described herein, some initial partitions, for example 100 partitions, are generated, amplified, and detected with the results yielding a preliminary analysis of how many partitions are positive or negative. This ratio of positive vs negative partitions provides an initial sample concentration. Based upon that result, any future partitions created from that sample can be adjusted. For example, one approach would be to vary the partition size as follows:
Assuming the starting concentration of an unknown sample is 10,000,000 target amplicons per 20 microliters, the following method can be used:
(a) Take 100 initial partitions at 1 nanoliter partition size from the starting sample theoretically containing 500 target copies for each partition, such that statistically all partitions would be positive. With reagents in a separate reservoir on the device, the system automatically combines the sample with reagents before amplification and detection. The calculated target concentration of the results based upon Poisson statistics would yield no result:

**Table 4**

| Theoretical # targets partitioned across 100 partitions (1 nL) | Estimated quantity | Standard Deviation | % CV |
|---|---|---|---|
| 50,000 | Unknown | N/A | N/A |

(b) Based upon the 100% positive partition/total partition result, the system takes a second set of 100 partitions at 1 picoliter partition size from the same starting sample. Due to its small size, each partition would therefore yield approximately 0.5 copies per partition. Poisson estimation would give the following results:

**Table 5**

| Theoretical # of targets partitioned across 100 partitions (1 pL) | Estimated quantity | Standard Deviation | %CV |
|---|---|---|---|
| 50 | 50.26 | 3.93 | 7.8% |

(c) The user would then proceed using only 0.1001 microliter (100 nanoliters for the first set of 100 one nanoliter partitions and 100 picoliters for the second set of 100 one picoliter partitions) of the original 20 microliter sample volume for quantitation, i.e., 0.5% of the sample volume. Using such low volumes has the added benefit of saving the starting sample for other applications such as next generation sequencing, sample banking for future applications, etc.
(d) Although it is possible that the system can continue to quantify the remaining sample, it may not be needed if the 0.101 microliter was sufficiently precise to perform the calculations.

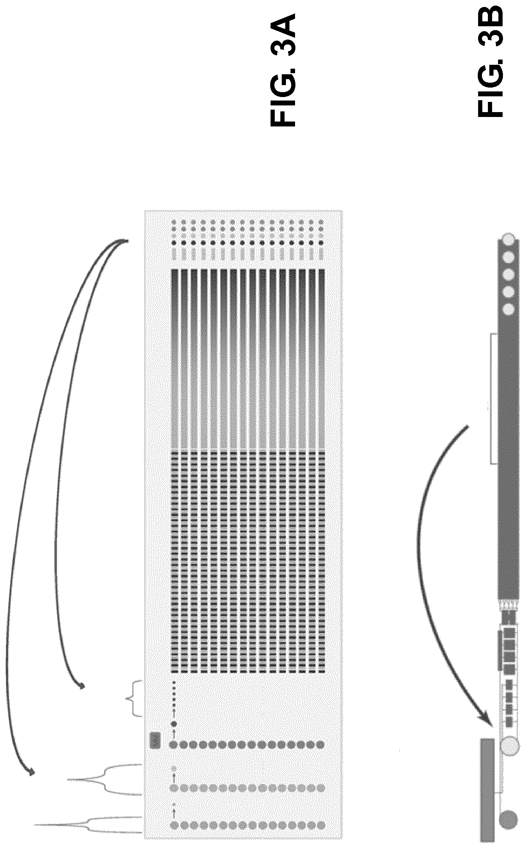

The number of partitions between the two sets does not need to be the same; this can be adjusted as the calculations will adjust according to the number of partitions. Moreover, the number of sets required to ultimately get to the optimal Poisson distribution is not limited to 2 as discussed hereinabove, but rather as necessary until the sample is acceptably quantified. This method allows the system to automatically partition an initial set, evaluate quantitation accuracy, determine and carry out a dilution, if needed, for retesting of the same sample (Fig. 3A). The entire workflow enables complete automation without user intervention. Software algorithms can determine the next steps based upon the results. The user can simply specify the level of standard deviation or CV desired and the system will repetitively dilute a portion of the sample until that desired precision is achieved (Fig. 3B).

Another approach would be to dilute the sample between each set. Assuming the same scenario as above, the starting concentration of an unknown sample is 10,000,000 target amplicons per 20 microliters. The following method can be used:
(a) 100 initial partitions at one nanoliter partition size from that starting sample would theoretically contain 500 target copies for each partition meaning that statistically all partitions would be positive. The calculated target concentration of the results based upon Poisson statistic would yield no result:

**Table 6**

| Theoretical # of targets partitioned across 100 partitions (1 nL) | Estimated quantity | Standard Deviation | %CV |
|---|---|---|---|
| 50,000 | Unknown | N/A | N/A |

(b) Based upon the 100% positive partition/total partition result, the system would perform an initial 1,000 fold sample dilution using a water reservoir on board the consumable, i.e., one nanoliter of sample diluted with 999 nanoliter of water. Alternatively, instead of a single 1,000 fold dilution as shown in this case, a serial dilution can be performed to achieve the 1:1000 fold dilution.
(c) Take a second set of 100 partitions at 1 nanoliter partition size from the 1:1000 diluted sample. Due to its small size, each partition would therefore yield approximately 0.5 copies per partition. Poisson estimation would give these results:

**Table 7**

| Theoretical # of targets partitioned across 100 partitions (1 pL) | Estimated quantity | Standard Deviation | %CV |
|---|---|---|---|
| 50 | 50.26 | 3.93 | 7.8% |

(d) The user would then be done using only 0.101 microliter (100 nanoliters for the first set of 100 1 nanoliter partitions and 1 nanoliter for the second set of 100 partitions from the fold dilution) of the original 20 microliter sample volume for quantitation, i.e., 0.5% of the sample volume. Using such low volumes has the added benefit of saving the starting sample for other applications such as next generation sequencing, sample banking for future applications, etc.
(e) Although it is possible that the system can continue to quantify the remaining sample, it may not be necessary if the 0.101 microliter was sufficiently precise to perform the calculations.

As described above, the partition sizes between the two sets do not need to be the same; this can be adjusted as the calculations will adjust according to the number of partitions. Likewise, the number of sets required to ultimately achieve the optimal Poisson distribution is not limited to two, but rather as necessary until the sample is acceptably quantified. Finally, as described in reference to the method above, this method allows the system to automatically partition an initial set, evaluate quantitation accuracy, determine and perform a dilution if needed for retesting of the same sample. In comparison, the following steps would be the typical method users of today's digital PCR system (assuming the same scenario as above, with a starting concentration of an unknown sample of 10,000,000 target amplicons per 20 microliters):
(i) Manually aliquot an initial volume of sample for preliminary quantitation. Typically with standard pipettors and workflows, 1-2 microliter is typically used for this step.
(ii) Using a spectrophotometer or fluorometer, determine approximate nucleic acid concentration in that 1-2 microliter volume.
(iii) Calculate the total nucleic acid concentration from the sample.
(iv) Estimate the concentration of the target amplicon from the total nucleic acid concentration, i.e., estimating between 1,000,000 to 100,000,000 target amplicons for example.
(v) Using a dPCR system giving 20,000 partitions, perform a 1,000 fold serial dilution using 1 microliter of sample.
(vi) Setup a PCR reaction with the diluted sample.
(vii) Work through the digital PCR workflow.
(viii) At the end of the workflow, analyze the results to determine the original sample concentration. Given the error associated with the pre-quantitation step (iv), it is possible that the standard deviation or CV is not within acceptable levels. If so, the process must be repeated from step (v) with a more accurate dilution based upon the current results.

The sample volumes consumed in this current workflow is 2-3 microliters. This is significantly more than envisioned in the present disclosure.

In comparison to these three approaches, the first two are envisioned in the present disclosure. The benefits of this would (1) allow the user to walk away from having to pre-quantify their samples through another method such as a spectrophotometry/ fluorometry/ electrophoresis, (2) reduce reagent cost since the system will optimized the minimum amount of sample needed to quantify the sample, (3) reduce consumables cost since the system can perform multiple rounds of quantitation using the same consumable, and (4) conserve sample so that additional sample isn't needed for a secondary run if a dilution is needed to get into the dynamic range of the digital PCR system. Additionally, digital microfluidics technology maximizes quantitation accuracy by minimizing the error inherent in manual pipetting methods. Moreover, the final quantitation determination for the sample in today's digital PCR system is a manual process that required the user to calculate the original starting sample based upon dilution while setting up the PCR reaction volume. Using digital microfluidics, since this system performs the necessary calculations to adjust for any dilution done automatically, the original starting sample concentration can be automatically determined. Another application utilizing automatic dilutions using digital microfluidics is for multiplexing applications using melting curve [6] or high resolution melting [7]. This will be discussed in further detail in the next section.

### Example 2. Quantitative multiplexing using a single fluorophore

Today's digital PCR and real-time PCR systems are limited in multiplexing by fluorophores - typically up to 6 targets. However, the partitioning of individual targets so that ideally one partition only has one target amplicon in digital PCR allows for the ability to quantitatively multiplex greater than two targets. What was once a qualitative analysis through melting curve or high resolution melting (HRM) in real-time PCR becomes a quantitative analysis in digital PCR. Using HRM or melt curve, quantitative multiplexing to the sensitivity and precision of digital PCR can be attained in targets over two and into the hundreds of targets [1, 2, 8]. There have been some examples of real-time PCR assays that note the ability of using melt curve to better discriminate multiple amplicons but the level of digital PCR-like quantitation is not possible (e.g. Seegene TOCE Technology and Roche SeptiFast kit). No commercial digital PCR system today takes advantage of quantitative multiplexing using melt curve or HRM. While today's multiplexing using melt curve/HRM still depends on different fluorophore signals, with digital PCR quantitative multiplexing is possible with a single fluorophore either as an intercalating DNA binding dye like SYBR Green or probe-based chemistries like Molecular Beacons.

Novel mutations can be detected and verified using this method. As discussed herein, a typical melt profile in real-time PCR is an average melt profile of all the species within the sample. This does not allow for sensitive discrimination between the target and a slight variant. Since digital PCR separates the target and the variant into different partitions, the melting profile for each partition is distinct and can be discriminated from each other, allowing identification of unknown variants. Applications of this approach include viral mutations research (such as HIV) where a patient being treated can develop a resistance due to viral mutations. Being able to monitor patients during treatment with assays that can identify when new viral mutations occur would be valuable from a research perspective but clinically relevant as well. This embodiment also allows for the isolation of variants for validation in downstream analyses such as sequencing.

Digital microfluidics has been used to perform high resolution melting [9]. In the present disclosure, HRM is provided for quantitation, specifically quantitative multiplexing in digital PCR.

Multiplexing has been in continuous demand over the years from users of real-time PCR and digital PCR. Yet the ability to discriminate fluorophores has only allowed for a small degree of multiplexing. Nanostring® is one technology that has introduced a novel way to multiplex up to 800 targets at a time; however the sensitivity is not quite comparable to PCR-based methodologies with cost and throughput being limited. With the approach described herein, it would be possible to multiplex hundreds of targets without the difficulties typically associated with multiplexing since in digital PCR, partitioning reduces multiplexing to singleplexes through limited dilution of the targets and stochastic distribution of various targets in various partitions not necessarily in the same partition (see FIG. 3).

With this level of quantitative multiplexing, this could finally address the market's quantitative multiplexing needs [1, 2]; take for example the sepsis diagnostic market, microbiome analysis, and genetic biomarker panels that could benefit with PCR sensitivity but multiplexing upwards of 2 to hundreds of targets.

Melting curve and HRM analysis in real-time PCR is not equivalent to that performed by digital PCR. In digital PCR, since each partition contains only a single template as the original starting material before amplification, all of the amplicon products for each partition after PCR amplification are homogenous. This homogeneity allows for better melt curve/HRM data to discriminate against other partitions [1, 2, 8]. In real-time PCR, the heterogenous sample of multiple targets after amplification will still remain heterogenous and thus the melt curve/HRM will be a reflection of that heterogeneity. This makes discriminating multiple targets via melting curve/HRM difficult and thus rely on fluorophores to perform multiplexing.

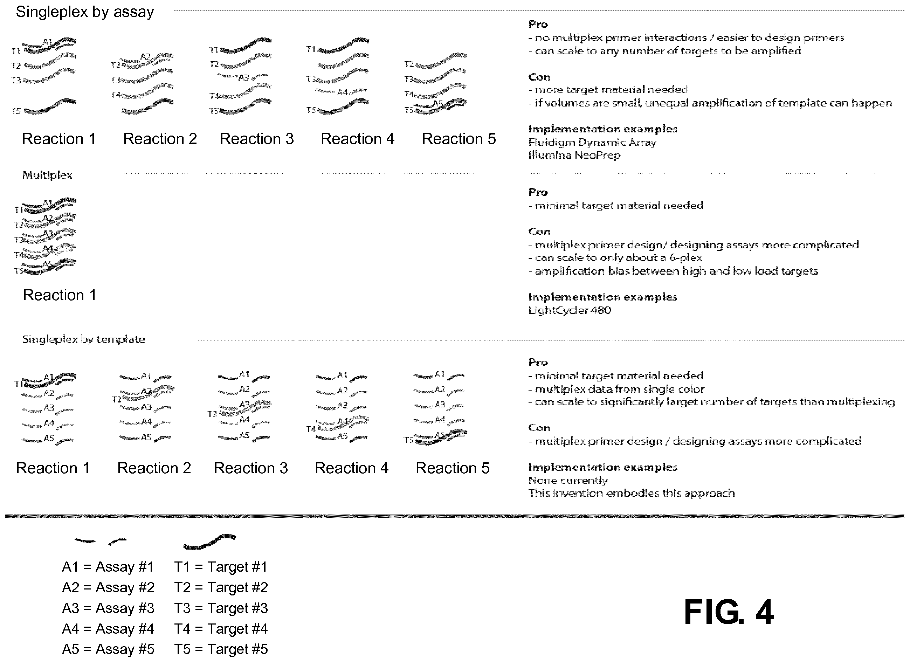

FIG. 4 illustrates this discussion. The melt curve/HRM of a heterogeneous real-time PCR sample with two targets yields a curve reflecting the melting profiles of both amplicon products. In contrast, a digital PCR melt curve will be generated for each partition that only has one target template. In the real-time PCR melting profile, it is possible to discern 2 peaks but it would not be possible to quantify how many templates were responsible for each peak. And as more targets are added to a multiplex panel, the melt profiles can be almost indistinguishable especially in cases of mutation detection where a single base pair change has very subtle changes in melt curve (e.g. SNP analysis). Analysis of melt curve/HRM of a heterogeneous real-time PCR sample ultimately requires the user to alter the hybridization probe chemistries, e.g., using Molecular Beacons, TaqMelt, dual-oligo hybridization probes, etc. In contrast, using digital PCR, counting the number of peaks and sorting those peaks by unique melting profiles could conclude that there were two products each with two template partitioned across 4 partitions.

Moreover, using real-time PCR, all targets are in the same reaction vessel. This approach requires different signals/fluorophores to be available to highlight and quantify the targets of interest. Today's limit on the number of fluorophores considering the spectral overlap does not lend itself to multiplexing greater than six targets. Perhaps future fluorophores with smaller spectral profiles will allow for high degrees of multiplexing using real-time PCR. In certain embodiments using the methods described herein, all of the targets are isolated into individual partitions and therefore only one signal is required to identify which target is in each partition. However, signal intensity alone as an endpoint PCR signal exemplified in today's digital PCR systems is not sufficient enough to identify which target is in each partition. Employing nucleic acid dissociation properties through a melt profile will allow for target identification. And since each partition ideally has one starting template, simply counting the number of positive partitions grouped by similar melting profiles would allow for quantitative multiplexing. The present disclosure combines the advantages of sample partitioning with melting profile analysis to achieve this.

Certain embodiments allow quantitative multiplex with intercalating DNA-binding dyes. Quantitative multiplexing in real-time PCR today is done through using multiple fluorophores. Over the years many users have tried to reduce cost by using intercalating DNA-binding dyes in multiplex assays. These assays are often not quantitative but often to identify present or absence of targets such as SNP analysis or infectious agent identification [10, 11]. With the accuracy and precision found in partitioning targets (e.g. digital PCR) and melting profile analysis merged in the present disclosure, quantitative multiplexing can be attained using cost effective intercalating DNA-binding dyes.

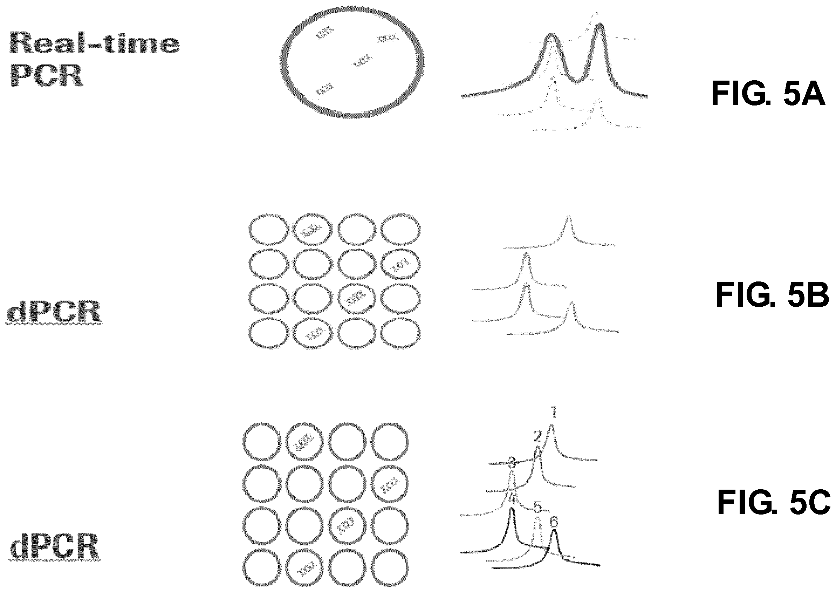

If multiple signals are used, such as fluorophores common to real-time PCR, this will increase the number of targets that can be multiplexed. As shown in Fig. 5A, in real-time PCR, a single melting profile is detected, representing a combined melting profile for all amplicons in the sample. In contrast, in Fig. 5B, dPCR enables the detection of a distinct melting profile for each partition. As illustrated in Fig. 5C, should two different targets share the same melting profile, such as the melt profile three vs four, or two vs five, or one vs six, each can be identified based upon the fluorophore color. Thus, if a combination of four fluorophores and if 30 targets can be identified with 30 distinct melting profiles for each fluorophore, a multiplexed panel could be used to identify 120 targets (30 x 4).

In addition, quantitative multiplexing as shown can be expanded in the number of targets. A recent publication has shown that with sufficient sample partitioning, a 92 multiplex assay is possible [1]. The difficulty in implementation as described in this publication is that the sample would need to be diluted proportionally depending on how many targets and of each target so that only 1 target template is in 1 partition. The present disclosure has described the use of dynamic partitioning based upon the sample concentration. Using dynamic partitioning as described, this would allow for implementation of a quantitative multiplex assay.

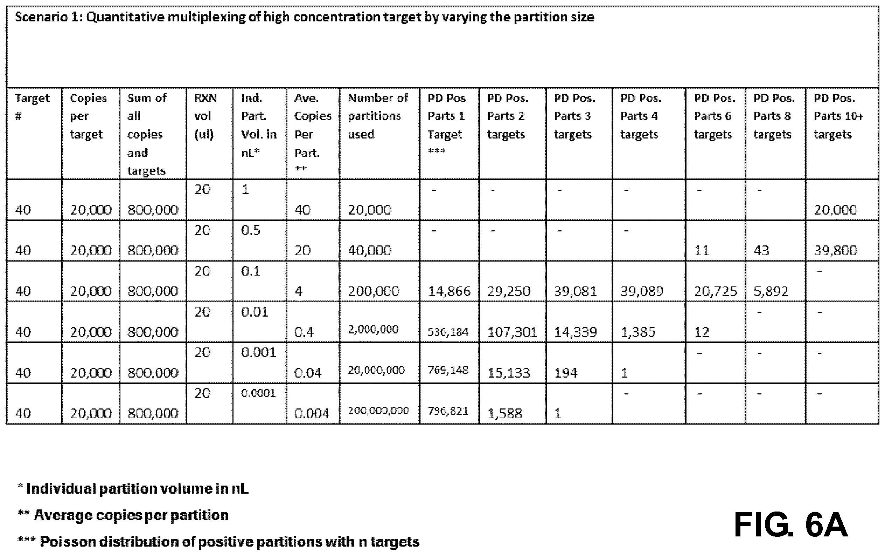

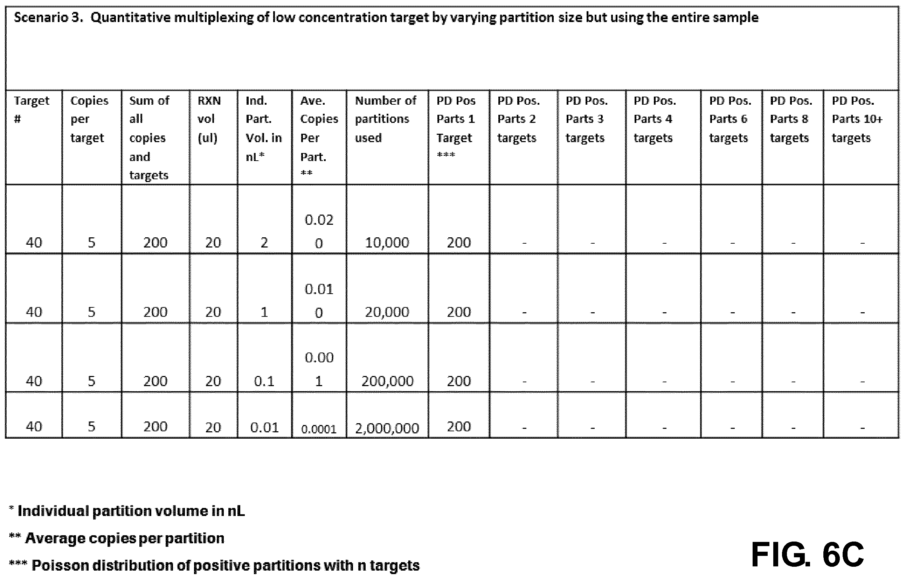

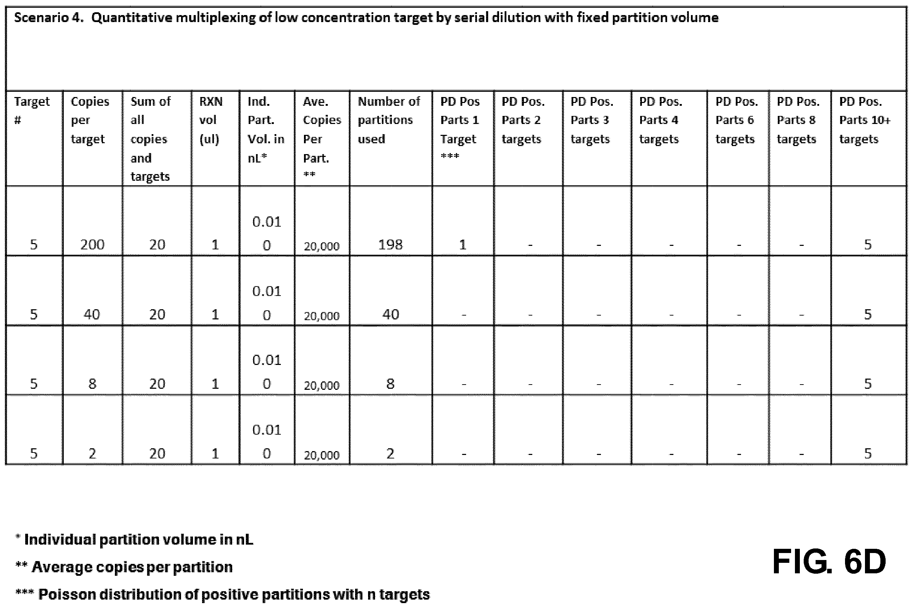

Figs. 6A-6D illustrate 4 scenarios describing quantitative multiplexing with and without dynamic partitioning with samples high in target concentration and low in target concentration. In Scenario 1 of Fig. 6A, assuming the number of targets to be 40 with, on average, 20,000 targets each (e.g., as within studies of gene expression, microbiome analysis, SNP analysis, etc.), in a 20 microliter reaction volume, there would be approximately 800,000 targets. With all the partitions having 10+ targets, this would be analogous to a heterogeneous mixture like in real-time PCR. As the partition volume is reduced, the Poisson distribution shows that more and more partitions will contain fewer and fewer targets, and eventually, 99.6% (796,821/800,000) of all targets will be distributed 1 target per partition. This allows for the homogenous mixture within each partition after amplification and melting curve analysis to be very discernable and identifiable as to which of the 40 targets is in that particular partition.

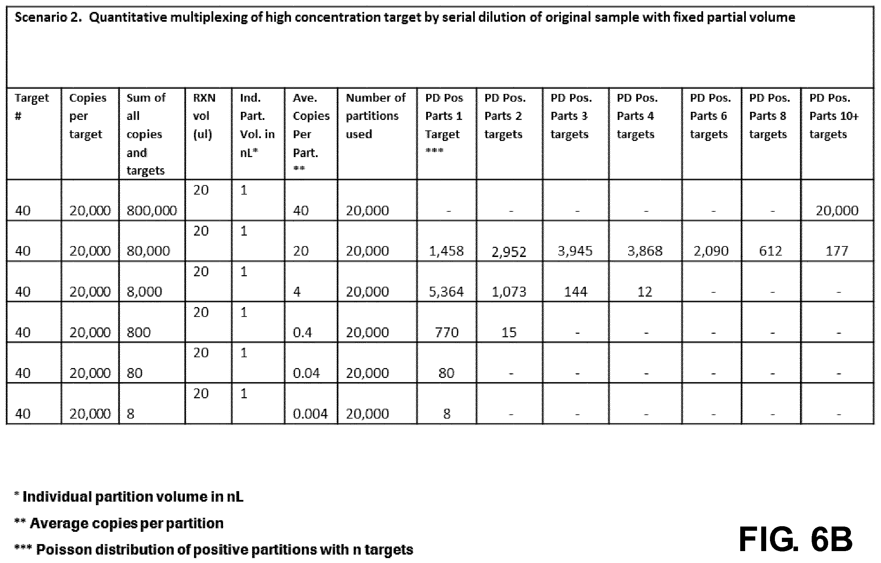

Certain embodiments will allow for varying of the partition volume since splitting and merging of partitions can be easily done. In Scenario 2 of Fig. 6B, assuming the same conditions as in Scenario 1, there would be approximately 800,000 targets. Instead of varying partition volume, the sample will initially be sampled using a dynamic partitioning workflow with an initial set of 100 partitions. Showing all 100 partitions to be positive, a 10-fold dilution can be employed. Quantitative multiplexing at this level would be difficult as the positive partitions with 2 or more targets per partition would be a heterogeneous sample and thus not accurately definable. However further dilution would show a Poisson distribution where 96% of positive partitions have only 1 target per partition (770/800). This would then allow for homogenous melt profiles and aid in identification and quantitation for each different target. Certain embodiments will allow for an initial analysis of the sample, followed by dynamic partitioning and dilution of the same sample to eventually achieve 1 target per partition and thus quantitative multiplexing by melt profiles. It will also be possible that should more than 1 target per partition exist, such as 2 targets, the melting profile can still be distinct enough from either target alone to allow identification and incorporating those partitions into the sample quantitation.

Scenario 3 of Fig. 6B , illustrates an example of low target copy number where high level of multiplexing is desired. Sepsis testing, low viral load screening (e.g. HIV, HBV, HCV, CMV, EBV, respiratory panels, etc.) as seen in blood screening or routine clinical diagnostics, and viral latency studies are possible applications. Assuming 40 different targets, each with on average 5 copies each in 20 microliters, a total of 200 targets need to be quantified. Given a high ratio of the number of partitions to low number of targets, the impact of the partition size is not significant as shown in the observed Poisson distribution s. Therefore any partition size illustrated would accurately allow for quantitative multiplexing. This is the same conclusion if dynamic partitioning was used.

Multiplexing through real-time PCR has an additional disadvantage when it comes to quantifying multiple targets that are drastically different in initial concentration. If a given target has only 10 copies in the reaction while another target has 10⁷ copies, in theory the dynamic range of a real-time PCR system should be able to quantify both. However it has been extensively discussed since the advent of real-time PCR that amplification efficiency are affected when high and low targets are amplified together [12]. Table 8 (below) shows that when 2 targets of vast differences in concentrations are multiplexed today, the amount of product from target 2 will greatly over take the reaction depleting the Taq polymerase and dNTPs much faster affecting the amplification efficiency of target 1 making accurate quantitation difficult. The result is that users of real-time PCR systems need to separate their assays and run quantification assays for each target in separate wells.

**Table 8**

| **Number of amplicons at the end of each cycle assuming 100% amplification efficiency** | | |
|---|---|---|
| **Cycle #** | **Target 1** | **Target 2** |
| Cycle 1 | 10 | 10,000,000 |
| Cycle 2 | 20 | 20,000,000 |
| Cycle 3 | 40 | 40,000,000 |
| Cycle 4 | 80 | 80,000,000 |
| Cycle 5 | 160 | 160,000,000 |
| Cycle 6 | 320 | 320,000,000 |
| Cycle 7 | 640 | 640,000,000 |
| Cycle 8 | 1,280 | 1,280,000,000 |
| Cycle 9 | 2,560 | 2,560,000,000 |
| Cycle 10 | 5,120 | 5,120,000,000 |
| Cycle 11 | 10,240 | 10,240,000,000 |
| Cycle 12 | 20,480 | 20,480,000,000 |
| Cycle 13 | 40,960 | 40,960,000,000 |
| Cycle 14 | 81,920 | 81,920,000,000 |
| Cycle 15 | 163,840 | 163,840,000,000 |
| Cycle 16 | 327,680 | 327,680,000,000 |
| Cycle 17 | 655,360 | 655,360,000,000 |
| Cycle 18 | 1,310,720 | 1,310,720,000,000 |
| Cycle 19 | 2,621,440 | 2,621,440,000,000 |
| Cycle 20 | 5,242,880 | 5,242,880,000,000 |
| Cycle 21 | 10,485,760 | 10,485,760,000,000 |
| Cycle 22 | 20,971,520 | 20,971,520,000,000 |
| Cycle 23 | 41,943,040 | 41,943,040,000,000 |
| Cycle 24 | 83,886,080 | 83,886,080,000,000 |
| Cycle 25 | 167,772,160 | 167,772,160,000,000 |
| Cycle 26 | 335,544,320 | 335,544,320,000,000 |
| Cycle 27 | 671,088,640 | 671,088,640,000,000 |
| Cycle 28 | 1,342,177,280 | 1,342,177,280,000,000 |
| Cycle 29 | 2,684,354,560 | 2,684,354,560,000,000 |
| Cycle 30 | 5,368,709,120 | 5,368,709,120,000,000 |
| Cycle 31 | 10,737,418,240 | 10,737,418,240,000,000 |
| Cycle 32 | 21,474,836,480 | 21,474,836,480,000,000 |
| Cycle 33 | 42,949,672,960 | 42,949,672,960,000,000 |
| Cycle 34 | 85,899,345,920 | 85,899,345,920,000,000 |
| Cycle 35 | 171,798,691,840 | 171,798,691,840,000,000 |
| Cycle 36 | 343,597,383,680 | 343,597,383,680,000,000 |
| Cycle 37 | 687,194,767,360 | 687,194,767,360,000,000 |
| Cycle 38 | 1,374,389,534,720 | 1,374,389,534,720,000,000 |
| Cycle 39 | 2,748,779,069,440 | 2,748,779,069,440,000,000 |
| Cycle 40 | 5,497,558,138,880 | 5,497,558,138,880,000,000 |

In certain embodiments, given that the optimal concentration can be empirically reached through dynamic partitioning as described above and with melt profile analysis, targets that are vastly different from one another can be accurately quantified. Competition for Taq polymerase and dNTPs reagents in the reaction are removed through partitioning as there is only 1 target per partition. Quantitative multiplexing without concern for varying target concentrations can be employed from the single sample.

Digital microfluidics facilitates this multiplexing when combined with digital partitioning of sample and melt profile analysis. As each individual partition containing a single template is passed through a temperature zone (e.g. a melt curve protocol) the melting of double stranded DNA can be observed. Various technologies can be employed to detect these passing partitions. Traditional fluorophore chemistries (e.g. Molecular Beacons, TaqMelt, Hybridization probes) can be used. Another approach is to use electrochemical intercalating dyes or electrochemical Molecular Beacon-like probes that bind to DNA [13-23]. Through a redox reaction, the conductance of the partition can be measured to determine if PCR amplicon exists. As these partitions are passed through a temperature gradient, changes in conductance can be measured directly by digital microfluidic technology when the PCR amplicon dissociates.

Another application for quantitative multiplexing in digital PCR is determining genetic linkages. One example is bacterial drug resistance testing in sepsis. Multiplex testing in real-time PCR can identify multiple infectious agents with a sample. It can also be used to identify a drug resistance gene. There are clinical implications in knowing which infectious agent possesses the drug resistance gene. However in real-time PCR since all the targets are within a single reaction vessel, it is not possible to determine which infectious agent possesses the drug resistance gene. In digital PCR, the genomes of each infectious agent are isolated away from each other. Therefore in the same multiplex assay used in real-time PCR, it will be possible to identify which infectious agent possesses the drug resistance gene since PCR product will colocalize to the same partition. Ultimately these partitions can be sequestered on the device and isolated for downstream application such as sequencing to confirm.

### Example 3. Resolving low or false positive partitions

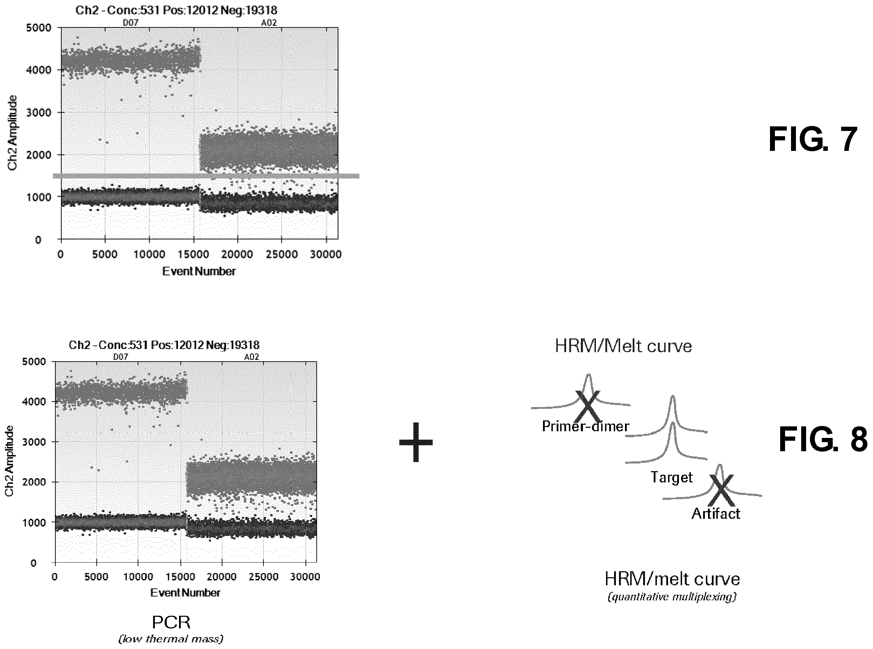

Being endpoint PCR, digital PCR results today are challenged with low or false positive partitions. Some have proposed that low false positives arise from poor assay design resulting in poor amplification, spontaneous hydrolysis of the probe, partition fragments, or true false positives due to contamination. The difficulty for the user is not being able to accurately determine if a positive partition is a low positive or a contamination event resulting in uncertainty about the value or relevance of the result. Significant time and resources have been spent to improve assays to reduce low positive partitions through increasing amplification cycles (thus reducing turnaround time) and/or redesign assay primers and probes. Although it has been argued that low positive partitions are infrequent and have little consequence on an clinical or research application, this is not necessarily true if the user is looking at rare events as experienced in HIV latency studies [24-27]. A few low positives when only a few positive partitions are expected can have a significant impact on clinical or research applications. Fig. 7 shows a diagram illustrating this from Bio-Rad's QX200 analysis software. Events 0 to approximately 15,500 belong to one sample, while events 15,501 to 32,000 belong to a different sample. In sample 1, the user will see positive partitions around 4200 on the y-axis (this is the signal intensity per partition) and negative partitions around 1000. In sample 2, positive partitions around 2000 and negative partitions about 900. In both examples, there are partitions that do not fall within either the positive or negative groupings. These partitions are commonly referred to as "rain." The existence of rain affects the precision of results. In applications where the number of targets are expected to be low such as in low viral load for blood screening or clinical diagnostics, sepsis testing, viral latency studies, a few rain partitions amongst the already few positive partitions can represent a significant uncertainty. This uncertainty does not exist today in real-time PCR. Since all templates are amplified in a single vessel, an average readout is obtained masking any of this uncertainty. Melt profiles are often employed in real-time PCR to confirm whether the expected amplicons were created. For digital PCR users today, almost all samples have a certain amount of rain.

The field currently uses statistical algorithms to eliminate rain, mainly using means and standard deviations to establish a positive-negative partition calling threshold. The difficulty in this approach is that the same assay from day to day on sample to sample can vary, thus making positive partition determination difficult. Automating digital PCR becomes challenging as each sample would need to be manually reviewed to ensure proper determination of sample results.

Certain embodiments use melting profiles to resolve issues associated with rain. Using HRM or melt curve data, an added layer of information about the partition can be obtained to better discriminate whether the low positive partition is either a different product, or high background fluorescence. With a melt curve profile for each partition, the user can better determine if the correct amplicon was created and thus facilitate automated partition. Rain from primer-dimer or artifact amplification can be disregarded while low amplification efficiency is included (see Fig. 8).

### Example 4. Assay validation for clinical application

Digital PCR is making strong headwind into the quantitative PCR market. The inherent sensitivity it offers over real-time PCR due to employing Poisson statistics instead of amplification efficiency from cycle to cycle as well as synthetic enrichment of sample due to partitioning has positioned itself into a niche application of low viral load quantitation and rare mutation detection where its precision allows for better diagnostic confidence when sample material can be limiting. However due to the issues associated with rain, validation of assays developed on digital PCR systems has been a challenge.

Through digital microfluidics, the isolation of positive partitions allows users to isolate particular partitions (e.g. positive vs negative partitions, rain partitions vs high signal partitions) to perform downstream analysis such as sequencing to validate the assay performance. The current systems on the market with droplet partitions or immobilized partitions make sorting and isolating specific partitions challenging. Digital microfluidics can easily handle this task since it controls every partition movement. Reservoirs on the consumable have been designed to hold partitions of interest to be collected for downstream applications (see Fig. 2C). At the far right of Fig. 2C, there are reservoirs (e.g., (222)) that can hold various partitions. The user can isolate only certain positive partitions with a specific melting profile. The number of reservoirs can be flexibly changed depending on the needs of the user.

### Example 5. Fast workflow, turnaround time and high sample throughput

As the applications for next generation sequencing are growing, one challenge users have is ensuring that the nucleic acid sample quality is appropriate. The degree of template fragmentation, purity of sample, and concentration of template can significantly affect the results. Capillary electrophoresis, spectrophotometry and fluorometry technologies such as the Agilent Bioanalyzer and Thermo Fisher NanoDrop and Qubit are often used in determining if a sample is sufficiently prepared for the next generation workflow. Some users have started to use real-time PCR instead since the ultimate determination of sample quality is whether a sample can is amplifiable as next generation sequencing itself requires PCR during the library preparation step before sequencing. One limitation of next generation sequencing technology embodied by Illumina's MiSeq and HiSeq is the sensitivity to overclustering or underclustering the sample lanes. Overclustering results is inaccuracy as the clusters overlap while underclustering results in underperformance of sequence generation resulting in rerunning samples. Given the large discrepancy in time and resources in performing real-time PCR vs electrophoresis/spectrophotometry/fluorometry, many users are slow to adopt real-time PCR as a replacement to these other methods.

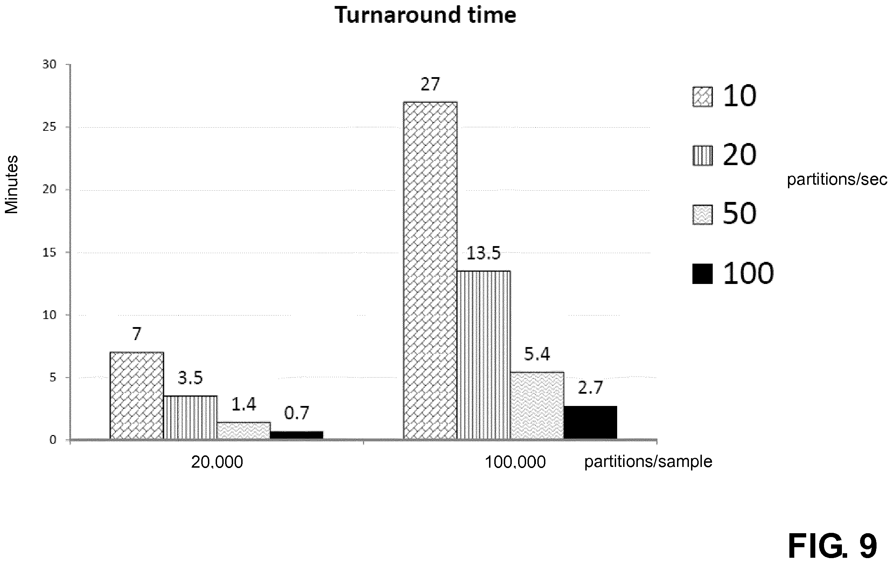

Certain embodiments will allow for significantly faster results as the entire workflow from partition generation, partition movement, serial dilutions if required, PCR, melting curve, and analysis is integrated into one system. Also, based upon the current state of digital microfluidic technology, the speed of partition processing can reduce the time to result from 45-60 mins down to less than 6 mins - making it similar in time to capillary electrophoresis, spectrophotometry and fluorometry techniques with the added benefits of library construction that can be directly transferred into next generation sequencing applications. Based upon current digital microfluidic technology, partitions can be generated and moved at high rates, e.g., up to 100 partitions/second[3]. In certain embodiments, since partitions are moving to various temperature zones, PCR amplification can happen significantly faster than real-time PCR. Many of today's real-time PCR systems such as the Thermo QuantStudio 6, Bio-Rad CFX96, Agilent MX3005P, Roche LightCycler have samples stationary while the temperature around the sample is heated and cooled. The heating and cooling rates are substantially limiting accounting for approximately 75% of the time for a typical PCR run of 40-60 mins. Even today's digital PCR systems such as the Bio-Rad QX200, Thermo QuantStudio 3D, Formulatrix Constellation, Fluidigm BioMark, Raindance Raindrop have designed the PCR thermal cycling step around the same principle. Unfortunately the additional oil/plastic/metal surrounding the partition requires thermocycling a larger mass and thus longer ramp rates and temperature hold times. In certain embodiments of partition movement to temperature zones eliminates the delays from ramping up and ramp down temperatures resulting in significant time savings. Since each partition is moving in sequence, there is low thermal mass so that reaching the appropriate temperature is fast. The table shown in FIG. 9 illustrates the sample turnaround time using digital microfluidic technology. Assuming partitions are moved across the consumable at 10 partitions/sec, 20,000 partitions will be generated, PCR amplified, and melt curve profiled in 7.0 minutes. If partition movement can be increased to 100 partitions/sec as described [3], the turnaround time will drop to 0.7 minutes.

If 100,000 partitions are needed for quantitating higher volume samples, at 100 partitions/sec using 20 lanes, samples can be completed in 2.7 mins.

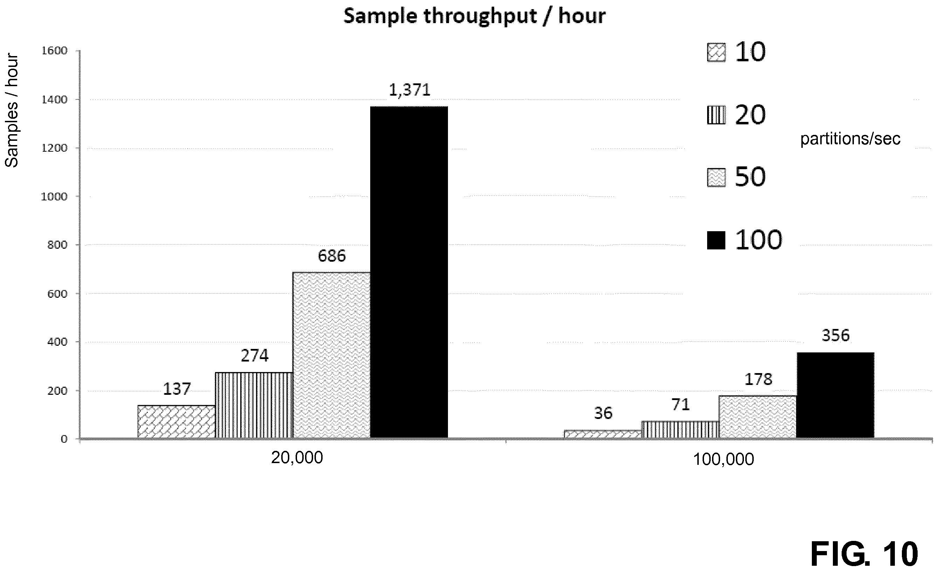

These fast turnaround time will allow for higher throughput. Fig. 10 shows a chart illustrating the sample throughput per hour. The assumption behind these calculations is based upon 16 samples having 20 lanes per consumable. If 40 lanes are used per sample, the sample throughput stays the same since the number of samples/consumable is reduced from 16 to 8 sample slots offsetting the 20 to 40 lanes gains from higher turnaround time. This high turnaround time would allow for nucleic quantitation in quantitative multiplexed fashion at speeds comparable to spectrophotometry/fluorometer.

Certain embodiments can also be used for library prep in the sequencing workflow (see Fig. 2A). The reagent reservoir for PCR can be setup to hold different assays. Each unique reagent (assay) can be split and combined with each sample before partition generation. This will allow for easy PCR setup if multiple assay panels are needed to be run against multiple samples. This can be applied to samples where various assay panels are run. Library construction for sequencing can also utilize certain embodiments.

Certain embodiments use many lanes for each sample. This facilitates faster processing of partitions which can be quite significant at 20,000 or much more. In the current design, it is set at 20 lanes but it can be anywhere from 2 or more lanes.

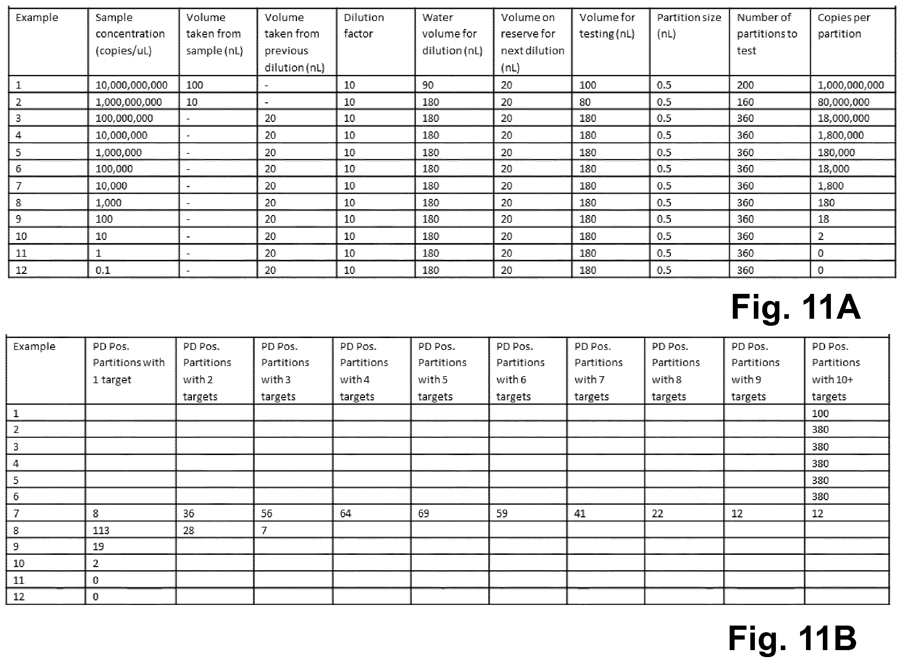

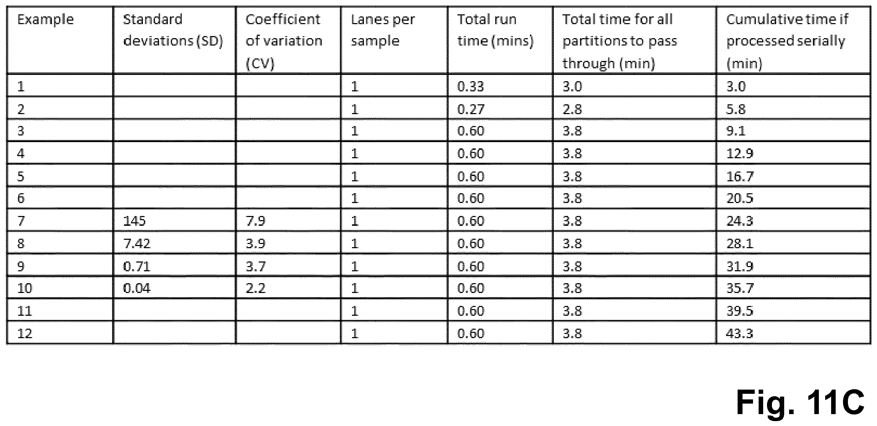

Assume partition movement across the partition at 10 partitions/sec, a sample concentration of 1¹⁰, partition size of 0.5 nanoliter, and serial dilution of 10X is performed, table in Figs. 11A-11C show how long it would take to process each successive dilution in serial order. Processing the first set of 200 partitions will require 3.0 minutes before the results are ready. If the first dilution in line 3 is performed, the 160 partitions there will take an additional 2.8 bring the total from the first 200 and second 160 to 5.8 minutes. Ultimately if each dilution is analyzed serially, by the time the system gets to a quantifiable range as defined by Poisson distribution, this would have taken 24.3 minutes. Ideally quantified results would be better with line 9 having a lower SD and CV which would take 28.1 minutes.

Since there are many lanes, it is possible to assign each lane for each dilution in the series so that all partitions are being processed in parallel. In this example, the system automatically performs all dilution series across 12 lanes. This would reduce the total time to results from 24.3 minutes to under 4 minutes. If the SD and CV are acceptable with the number of partitions used to quantify, the system can stop. However if a higher SD or CV is needed, the system can identify which dilution was optimal and assign all lanes to process partitions from that dilution step.

Similarly this approach of using each lane independently can be adapted to quantify multiplex targets. A multiplex assay can quantify targets with a wide range of quantities, one target being 100 copies/ul while another at 100,000,000,000 copies/ul; therefore, one dilution might be optimal for one target, a different dilution would be optimal for a different target. Different lanes can then be assigned to quantify each target. For example, Fig. 2D shows the area of the consumable where this can be implemented. Area 1-4 (215) are PCR reagent staging areas in which one or more PCR reagents are mixed with the partition present in that area. Area 1 can contain the 100 copies/ul dilution mixture while Area 2 can contain the 100,000,000 copies/ul dilution mixture. Both are mixed with PCR reagents in these areas. Once proper mixing of sample and PCR mix has occurred, the contents of Areas 1 and 2 are migrated via digital microfluidics successively into Areas 5 and 6, respectively. Area 5 will then proceed to dispense partitions into lane 1 or more, e.g., up to lane 10, while Area 6 will then dispense partitions into lane 11 or more, e.g., between lanes 11-20. Therefore, the same sample is being quantitated for different targets across different lanes. Even though the starting targets are drastically different, serial dilution of the sample in different chambers followed by the separation of partitions through PCR and melt profile in different lanes allows for quantitating sample at the appropriate target concentrations. Although it is technically possible to just dilute the entire sample so that the highest concentration target is diluted to the dynamic range of the consumable, that approach might generate a significant volume of partitions that would take a significant amount of time and reduce sample throughput.

### REFERENCES

1. Athamanolap, P., et al., Trainable high resolution melt curve machine learning classifier for large-scale reliable genotyping of sequence variants. PLoS One, 2014. 9(9): p. e109094.
2. Fraley, S.I., et al., Universal digital high-resolution melt: a novel approach to broad-based profiling of heterogeneous biological samples. Nucleic Acids Res, 2013. 41(18): p. e175.
3. Jagath, N.Y., et al., Droplet dispensing and splitting by electrowetting on dielectric digital microfluidics in MEMS 2014. 2014: San Francisco, CA.
4. Whale, A.S., et al., Comparison of microfluidic digital PCR and conventional quantitative PCR for measuring copy number variation. Nucleic Acids Res, 2012. 40(11): p. e82.
5. Shen, F., et al., Multiplexed quantification of nucleic acids with large dynamic range using multivolume digital RT-PCR on a rotational SlipChip tested with HIV and hepatitis C viral load. J Am Chem Soc, 2011. 133(44): p. 17705-12.
6. Huang, Q., et al., Multiplex fluorescence melting curve analysis for mutation detection with dual-labeled, self-quenched probes. PLoS One, 2011. 6(4): p. e19206.
7. Rouleau, E., et al., Quantitative PCR high-resolution melting (qPCR-HRM) curve analysis, a new approach to simultaneously screen point mutations and large rearrangements: application to MLH1 germline mutations in Lynch syndrome. Hum Mutat, 2009. 30(6): p. 867-75.
8. Yang, S., et al., Rapid identification of biothreat and other clinically relevant bacterial species by use of universal PCR coupled with high-resolution melting analysis. J Clin Microbiol, 2009. 47(7): p. 2252-5.
9. Eckhardt, A.E. and Z. Hua, High Resolution Melting Analysis on a Droplet Actuator. 2013, Google Patents.
10. Horvath, A., et al., A novel, multiplex, real-time PCR-based approach for the detection of the commonly occurring pathogenic fungi and bacteria. BMC Microbiol, 2013. 13: p. 300.
11. Staggemeier, R., et al., Multiplex SYBR(R) green-real time PCR (qPCR) assay for the detection and differentiation of Bartonella henselae and Bartonella clarridgeiae in cats. Rev Inst Med Trop Sao Paulo, 2014. 56(2): p. 93-5.
12. Markoulatos, P., N. Siafakas, and M. Moncany, Multiplex polymerase chain reaction: a practical approach. J Clin Lab Anal, 2002. 16(1): p. 47-51.
13. Kober, E.M., et al., Synthetic Routes to New Polypyridyl Complexes of Osmium(I1). Inorganic Chemistry, 1988. 27: p. 12.
14. Guo, L.H., M.Y. Wei, and H. Chen, Multiple DNA binding modes of a metallointercalator revealed by DNA film voltammetry. J Phys Chem B, 2006. 110(41): p. 20568-71.
15. Holmlin, R.E., E.D. Stemp, and J.K. Barton, Os(phen)2dppz2+ in Photoinduced DNA-Mediated Electron Transfer Reactions. J. Am. Chem. Soc., 1996. 118: p. 9.
16. Arkin, M.R., et al., Rates of DNA-mediated electron transfer between metallointercalators. Science, 1996. 273(5274): p. 475-80.
17. Maruyama, K., et al., Electrochemical characterization and DNA-binding property of dipyridophenazine complexes of osmium (II). Nucleic Acids Symp Ser, 2000(44): p. 59-60.
18. Sato, S. and S. Takenaka, Electrochemical DNA detection using supramolecular interactions. Anal Sci, 2012. 28(7): p. 643-9.
19. Syed, S.N., et al., Cyclic denaturation and renaturation of double-stranded DNA by redox-state switching of DNA intercalators. J Am Chem Soc, 2013. 135(14): p. 5399-407.
20. Takenaka, H., S. Sato, and S. Takenaka, Electrochemical detection of duplex DNA using intercalation-triggered decomplexation of ferrocene with beta-cyclodextrin. Electroanalysis, 2013. 25(8): p. 4.
21. Limoges, B., T. Defever, and D. Marchal, Method for electrochemically identifying target nucleotide sequences. 2011, Google Patents.
22. Defever, T., et al., Real-time electrochemical monitoring of the polymerase chain reaction by mediated redox catalysis. J Am Chem Soc, 2009. 131(32): p. 11433-41.
23. Defever, T., et al., Real-time electrochemical PCR with a DNA intercalating redox probe. Anal Chem, 2011. 83(5): p. 1815-21.
24. Strain, M.C., et al., Highly precise measurement of HIV DNA by droplet digital PCR. PLoS One, 2013. 8(4): p. e55943.
25. De Spiegelaere, W., et al., Quantification of integrated HIV DNA by repetitive-sampling Alu-HIV PCR on the basis of poisson statistics. Clin Chem, 2014. 60(6): p. 886-95.
26. Beliakova-Bethell, N., et al., Maraviroc intensification in patients with suppressed HIV viremia has limited effects on CD4+ T cell recovery and gene expression. Antiviral Res, 2014. 107: p. 42-9.
27. Henrich, T.J., et al., Low-level detection and quantitation of cellular HIV-1 DNA and 2-LTR circles using droplet digital PCR. J Virol Methods, 2012. 186(1-2): p. 68-72.
28. Chang, Y. H., et al. Integrated polymerase chain reaction chips utilizing digital microfluidics. Biomedical microdevices 8, 215-225 (2006).)

## Claims

1. A microfluidic device (100, 200) comprising an upper (106) and lower (101) substrate and a lateral plane (108) positioned between the upper and lower substrate, the lower substrate comprising an electrode array (104) configured to move a partition along the lateral plane, wherein the lateral plane includes a plurality of zones comprising, from a proximate to a distal end,
(a) a preparation zone (201) comprising (i) a sample loading zone (204. 210), (ii) a water reservoir (206, 212) and one or more reagent reservoirs (207) each in communication with the sample loading zone; and (iii) a partition generation zone (208);
(b) an amplification zone (202, 220) in thermal communication with one or more heating elements configured to subject the amplification zone to a thermal protocol for polymerase chain reaction (PCR) amplification, and
(c) a melt curve zone (203, 221) in thermal communication with one or more additional heating elements configured to subject the melt curve zone to a thermal gradient to generate a melting profile of an amplification product.

2. The microfluidic device of claim 1, wherein the sample loading zone (204, 210) comprises a plurality of sample loading regions (205, 211) each configured to accommodate a partition.

3. The microfluidic device of any one of claims 1 to 2, wherein the amplification zone (202, 220) is in thermal communication with one or more heating elements configured to increase the temperature in the amplification zone from a proximate to a distal end.

4. The microfluidic device of any one of claims 1 to 3, wherein the melt curve zone (203, 221) is in thermal communication with one or more additional heating elements configured to subject a partition migrating through the melt curve zone to a thermal gradient.

5. The microfluidic device of claim 4, wherein one or more of the amplification zone (202, 220) and the melt curve zone (203, 221) is in optical communication with a detection system adapted to detect an optical signal emitted from a partition positioned in the amplification zone.

6. The microfluidic device of any one of claims 1 to 5, wherein the preparation zone (201) further comprises a sample dilution staging zone (213) comprising a plurality of dilution chambers (214), each in communication with the water reservoir (206, 212).

7. The microfluidic device of any one of claims 1 to 6, wherein the preparation zone (201) further comprises a PCR reagent staging zone (215) comprising a plurality of staging chambers (216), each in communication with the one or more PCR reagent reservoirs (217).

8. The microfluidic device of any one of claims 1 to 7, wherein the partition generation zone (208) comprises a partition generation staging area (218) including a plurality of partition generation staging chambers (219).

9. The microfluidic device of any one of claims 2 to 8, wherein the preparation zone (201) further comprises a sample dilution staging zone (213) including a plurality of dilution chambers (214), wherein at least one dilution chamber of the plurality of dilution chambers is in communication with a sample loading region (205, 211) and the water reservoir (206, 212).

10. The microfluidic device of claim 9, wherein the preparation zone (201) further comprises a PCR reagent staging zone (215) comprising a plurality of staging chambers (216) each in communication with one or more PCR reagent reservoirs (217), wherein the at least one dilution chamber of the plurality of dilution chambers (214) is in communication with at least one staging chamber of the plurality of staging chambers.

11. A method of performing digital PCR on an electrowetting-based microfluidic device (100, 200) according to any one of claims 1 to 10, the method comprising the steps in the following order:
(a) adding a partition comprising a sample to a sample loading zone (204, 210) positioned on the device,
(b) adding a volume of water to the partition in the sample loading zone (204, 210) and thereby diluting the partition;
(c) adding a PCR reagent mixture to the partition in the sample loading zone (204, 210);
(d) partitioning the partition into a plurality of partitions, wherein the partitioning step is conducted in a partition generation zone (208) on the device;
(e) subjecting the plurality of partitions to a thermal protocol to generate one or more amplicon-containing partitions, wherein the subjecting step (e) is conducted in an amplification zone (202, 220) on the device; and
(f) subjecting the one or more amplicon-containing partitions to a thermal gradient and thereby generate a melting profile for each of the one or more amplicon-containing partitions, wherein the subjecting step (f) is conducted in a melt curve zone (203, 221) on the device;
(g) determining a sample concentration based on the results of step (f); and
(h) repeating steps (a)-(g) with one or more additional partitions comprising an adjusted partition size relative to the previous partition size to determine an adjusted sample concentration until an optimal Poisson distribution is achieved.

12. The method of claim 11, wherein a first set of partitions are subjected to steps (a)-(g) and the one or more additional sets of partitions are subjected to steps (a)-(g), wherein the sample in the one or more additional sets of partitions is serially diluted relative to the sample in the first set of partitions.

13. A method of performing a multiplexed digital PCR analysis on an electrowetting-based microfluidic device (100, 200) according to any one of claims 1 to 10, the method comprising the steps in the following order:
(a) adding a partition comprising a sample to a sample loading zone (204, 210) positioned on the device, wherein the sample comprises a plurality target sequences;
(b) adding a volume of water to the partition in the sample loading zone (204, 210) and thereby diluting the partition;
(c) adding a PCR reagent mixture to the partition in the sample loading zone (204, 210);
(d) partitioning the partition into a plurality of partitions, wherein the partitioning step is conducted in a partition generation zone (208) on the device;
(e) subjecting the plurality of partitions to a thermal protocol to generate one or more amplicon-containing partitions, wherein the subjecting step (e) is conducted in an amplification zone (202, 220) on the device;
(f) subjecting the one or more amplicon-containing partitions to a thermal gradient and thereby generate a melting profile for each of the one or more amplicon-containing partitions, wherein the subjecting step (f) is conducted in a melt curve zone (203, 221) on the device;
(g) detecting the presence and/or absence of each of the target sequences in the plurality of target sequences based on the melting profile for each of the one or more amplicons; and
(h) repeating steps (a)-(g) with one or more additional partitions comprising an adjusted partition size relative to the previous partition size until an optimal Poisson distribution is achieved.

14. The method of claim 13, wherein a first set of partitions are subjected to steps (a)-(g) and the one or more additional sets of partitions are subjected to steps (a)-(g), wherein the sample in the one or more additional sets of partitions is serially diluted relative to the sample in the first set of partitions.

15. The method of any one of claims 11 to 14, wherein, following step (e), each of the plurality of partitions comprises zero or one target sequence.

## Patentansprüche

1. Mikrofluidvorrichtung (100, 200), umfassend ein oberes (106) und ein unteres (101) Substrat und eine laterale Ebene (108), die zwischen dem oberen und dem unteren Substrat angeordnet ist, wobei das untere Substrat eine Elektrodenanordnung (104) umfasst, die zum Bewegen einer Partition entlang der lateralen Ebene ausgelegt ist, wobei die laterale Ebene eine Vielzahl von Zonen beinhaltet, umfassend, von einem proximalen zu einem distalen Ende,
(a) eine Präparationszone (201), umfassend (i) eine Probenbeladungszone (204, 210), (ii) einen Wasserbehälter (206, 212) und einen oder mehrere Reagenzbehälter (207), die jeweils in Verbindung mit der Probenbeladungszone stehen; und (iii) eine Partitionserzeugungszone (208);
(b) eine Amplifikationszone (202, 220) in thermischer Verbindung mit einem oder mehreren Heizelementen, die dazu ausgelegt sind, die Amplifikationszone einem thermischen Protokoll für eine Polymerasekettenreaktion (PCR) Amplifikation zu unterwerfen, und
(c) eine Schmelzkurvenzone (203, 221) in thermischer Verbindung mit einem oder mehreren zusätzlichen Heizelementen, die dazu ausgelegt sind, die Schmelzkurvenzone einem thermischen Gradienten zu unterwerfen, um ein Schmelzprofil eines Amplifikationsprodukts zu erzeugen.

2. Mikrofluidvorrichtung nach Anspruch 1, wobei die Probenbeladungszone (204, 210) eine Vielzahl von Probenbeladungsregionen (205, 211) umfasst, die jeweils zum Aufnehmen einer Partition ausgelegt sind.

3. Mikrofluidvorrichtung nach einem der Ansprüche 1 bis 2, wobei die Amplifikationszone (202, 220) in thermischer Verbindung mit einem oder mehreren Heizelementen steht, die zum Erhöhen der Temperatur in der Amplifikationszone von einem proximalen zu einem distalen Ende ausgelegt sind.

4. Mikrofluidvorrichtung nach einem der Ansprüche 1 bis 3, wobei die Schmelzkurvenzone (203, 221) in thermischer Verbindung mit einem oder mehreren zusätzlichen Heizelementen steht, die dazu ausgelegt sind, eine sich durch die Schmelzkurvenzone bewegende Partition einem thermischen Gradienten zu unterwerfen.

5. Mikrofluidvorrichtung nach Anspruch 4, wobei eine oder mehrere der Amplifikationszone (202, 220) und der Schmelzkurvenzone (203, 221) in optischer Verbindung mit einem Erfassungssystem stehen, das zum Nachweisen eines optischen Signals beschaffen ist, das von einer in der Amplifikationszone angeordneten Partition gesendet wird.

6. Mikrofluidvorrichtung nach einem der Ansprüche 1 bis 5, wobei die Präparationszone (201) ferner eine Probenverdünnungsabstufungszone (213) umfasst, die eine Vielzahl von Verdünnungskammern (214) umfasst, die jeweils mit dem Wasserbehälter (206, 212) in Verbindung stehen.

7. Mikrofluidvorrichtung nach einem der Ansprüche 1 bis 6, wobei die Präparationszone (201) ferner eine PCR-Reagenzabstufungszone (215) umfasst, die eine Vielzahl von Abstufungskammern (216) umfasst, die jeweils mit dem einen oder den mehreren PCR-Reagenzbehältern (217) in Verbindung stehen.

8. Mikrofluidvorrichtung nach einem der Ansprüche 1 bis 7, wobei die Partitionserzeugungszone (208) einen Partitionserzeugungsabstufungsbereich (218) mit einer Vielzahl von Partitionserzeugungsabstufungskammern (219) umfasst.

9. Mikrofluidvorrichtung nach einem der Ansprüche 2 bis 8, wobei die Präparationszone (201) ferner eine Probenverdünnungsabstufungszone (213) mit einer Vielzahl von Verdünnungskammern (214) umfasst, wobei mindestens eine Verdünnungskammer der Vielzahl von Verdünnungskammern in Verbindung mit einer Probenbeladungszone (205, 211) und dem Wasserbehälter (206, 212) steht.

10. Mikrofluidvorrichtung nach Anspruch 9, wobei die Präparationszone (201) ferner eine PCR-Reagenzabstufungszone (215) umfasst, die eine Vielzahl von Abstufungskammern (216) umfasst, die jeweils mit dem einen oder den mehreren PCR-Reagenzbehältern (217) in Verbindung stehen, wobei die mindestens eine Verdünnungskammer der Vielzahl von Verdünnungskammern in (214) Verbindung mit mindestens einer Abstufungskammer der Vielzahl von Abstufungskammern steht.

11. Verfahren zum Durchführen einer digitalen PCR auf einer Mikrofluidvorrichtung (100, 200) auf Elektrobenetzungsbasis nach einem der Ansprüche 1 bis 10, wobei das Verfahren die Schritte in folgender Reihenfolge umfasst:
(a) Hinzufügen einer eine Probe umfassenden Partition zu einer auf der Vorrichtung angeordneten Probenbeladungszone (204, 210),
(b) Hinzufügen eines Volumens von Wasser zu der Partition in der Probenbeladungszone (204, 210), und dadurch Verdünnen der Partition;
(c) Hinzufügen einer PCR-Reagenzmischung zu der Partition in der Probenbeladungszone (204, 210);
(d) Partitionieren der Partition in eine Vielzahl von Partitionen, wobei der Partitionierschritt in einer Partitionserzeugungszone (208) auf der Vorrichtung durchgeführt wird;
(e) Unterwerfen der Vielzahl von Partitionen einem thermischen Protokoll zum Erzeugen einer oder mehrerer Amplikon-enthaltender Partitionen, wobei der Unterwerfungsschritt (e) in einer Amplifikationszone (202, 220) auf der Vorrichtung durchgeführt wird; und
(f) Unterwerfen der einen oder der mehreren Amplikon-enthaltenden Partitionen einem thermischen Gradienten und dadurch Erzeugen eines Schmelzprofils für jede der einen oder der mehreren Amplikon-enthaltenden Partitionen, wobei der Unterwerfungsschritt (f) in einer Schmelzkurvenzone (203, 221) auf der Vorrichtung durchgeführt wird;
(g) Bestimmen einer Probenkonzentration auf der Grundlage der Ergebnisse von Schritt (f); und
(h) Wiederholen der Schritte (a)-(g) mit einer oder mehreren zusätzlichen Partitionen, umfassend eine angepasste Partitionsgröße in Bezug auf die vorherige Partitionsgröße, zum Bestimmen einer angepassten Probenkonzentration, bis eine optimale Poisson-Verteilung erreicht ist.

12. Verfahren nach Anspruch 11, wobei ein erster Satz Partitionen den Schritten (a)-(g) unterworfen wird und der eine zusätzliche Satz oder die mehreren zusätzlichen Sätze Partitionen den Schritten (a)-(g) unterworfen werden, wobei die Probe in dem einen zusätzlichen Satz oder den mehreren zusätzlichen Sätzen Partitionen seriell in Bezug auf die Probe im ersten Satz Partitionen verdünnt wird.

13. Verfahren zum Durchführen einer digitalen Multiplex-PCR-Analyse auf einer Mikrofluidvorrichtung (100, 200) auf Elektrobenetzungsbasis nach einem der Ansprüche 1 bis 10, wobei das Verfahren die Schritte in folgender Reihenfolge umfasst:
(a) Hinzufügen einer eine Probe umfassenden Partition zu einer auf der Vorrichtung angeordneten Probenbeladungszone (204, 210), wobei die Probe eine Vielzahl Zielsequenzen umfasst;
(b) Hinzufügen eines Volumens von Wasser zu der Partition in der Probenbeladungszone (204, 210), und dadurch Verdünnen der Partition;
(c) Hinzufügen einer PCR-Reagenzmischung zu der Partition in der Probenbeladungszone (204, 210);
(d) Partitionieren der Partition in eine Vielzahl von Partitionen, wobei der Partitionierschritt in einer Partitionserzeugungszone (208) auf der Vorrichtung durchgeführt wird;
(e) Unterwerfen der Vielzahl von Partitionen einem thermischen Protokoll zum Erzeugen einer oder mehrerer Amplikon-enthaltender Partitionen, wobei der Unterwerfungsschritt (e) in einer Amplifikationszone (202, 220) auf der Vorrichtung durchgeführt wird;
(f) Unterwerfen der einen oder der mehreren Amplikon-enthaltenden Partitionen einem thermischen Gradienten und dadurch Erzeugen eines Schmelzprofils für jede der einen oder der mehreren Amplikon-enthaltenden Partitionen, wobei der Unterwerfungsschritt (f) in einer Schmelzkurvenzone (203, 221) auf der Vorrichtung durchgeführt wird;
(g) Nachweisen der Anwesenheit und/oder der Abwesenheit jeder der Zielsequenzen in der Vielzahl von Zielsequenzen auf der Grundlage des Schmelzprofils jedes des einen oder der mehreren Amplikons; und
(h) Wiederholen der Schritte (a)-(g) mit einer oder mehreren zusätzlichen Partitionen, die eine angepasste Partitionsgröße in Bezug auf die vorherige Partitionsgröße umfassen, bis eine optimale Poisson-Verteilung erreicht ist.

14. Verfahren nach Anspruch 13, wobei ein erster Satz Partitionen den Schritten (a)-(g) unterworfen wird und der eine zusätzliche Satz oder die mehreren zusätzlichen Sätze Partitionen den Schritten (a)-(g) unterworfen werden, wobei die Probe in dem einen zusätzlichen Satz oder den mehreren zusätzlichen Sätzen Partitionen in Bezug auf die Probe im ersten Satz Partitionen seriell verdünnt wird.

15. Verfahren nach einem der Ansprüche 11 bis 14, wobei jede der Vielzahl von Partitionen nach Schritt (e) null oder eine Zielsequenz umfasst.

## Revendications

1. Dispositif microfluidique (100, 200) comprenant un substrat supérieur (106) et un substrat inférieur (101) et un plan latéral (108) positionné entre le substrat supérieur et le substrat inférieur, le substrat inférieur comprenant un réseau d'électrodes (104) configuré pour déplacer une partition le long du plan latéral, dans lequel le plan latéral inclut une pluralité de zones comprenant, d'une extrémité proximale à une extrémité distale,
(a) une zone de préparation (201) comprenant (i) une zone de chargement d'échantillon (204, 210), (ii) un réservoir d'eau (206, 212) et un ou plusieurs réservoirs de réactif (207), chacun en communication avec la zone de chargement d'échantillon ; et (iii) une zone de génération de partitions (208) ;
(b) une zone d'amplification (202, 220) en communication thermique avec un ou plusieurs éléments chauffants configurés pour soumettre la zone d'amplification à un protocole thermique pour une amplification par réaction en chaîne par polymérase (PCR), et
(c) une zone de courbe de fusion (203, 221) en communication thermique avec un ou plusieurs éléments chauffants supplémentaires configurés pour soumettre la zone de courbe de fusion à un gradient thermique pour générer un profil de fusion d'un produit d'amplification.

2. Dispositif micro fluidique selon la revendication 1, dans lequel la zone de chargement d'échantillon (204, 210) comprend une pluralité de régions de chargement d'échantillon (205, 211), chacune configurée pour accueillir une partition.

3. Dispositif microfluidique selon l'une quelconque des revendications 1 à 2, dans lequel la zone d'amplification (202, 220) est en communication thermique avec un ou plusieurs éléments chauffants configurés pour augmenter la température dans la zone d'amplification d'une extrémité proximale à une extrémité distale.

4. Dispositif microfluidique selon l'une quelconque des revendications 1 à 3, dans lequel la zone de courbe de fusion (203, 221) est en communication thermique avec un ou plusieurs éléments chauffants supplémentaires configurés pour soumettre à un gradient thermique une partition migrant à travers la zone de courbe de fusion.

5. Dispositif micro fluidique selon la revendication 4, dans lequel une ou plusieurs de la zone d'amplification (202, 220) et de la zone de courbe de fusion (203, 221) sont en communication optique avec un système de détection conçu pour détecter un signal optique émis par une partition positionnée dans la zone d'amplification.

6. Dispositif micro fluidique selon l'une quelconque des revendications 1 à 5, dans lequel la zone de préparation (201) comprend en outre une zone de rassemblement de dilutions d'échantillon (213) comprenant une pluralité de chambres de dilution (214), chacune en communication avec le réservoir d'eau (206, 212).

7. Dispositif microfluidique selon l'une quelconque des revendications 1 à 6, dans lequel la zone de préparation (201) comprend en outre une zone de rassemblement de réactifs de PCR (215) comprenant une pluralité de chambres de rassemblement (216), chacune en communication avec un ou plusieurs réservoirs de réactifs de PCR (217).

8. Dispositif microfluidique selon l'une quelconque des revendications 1 à 7, dans lequel la zone de génération de partitions (208) comprend une zone de rassemblement de générations de partitions (218) incluant une pluralité de chambres de rassemblement de générations de partitions (219).

9. Dispositif microfluidique selon l'une quelconque des revendications 2 à 8, dans lequel la zone de préparation (201) comprend en outre une zone de rassemblement de dilutions d'échantillon (213) incluant une pluralité de chambres de dilution (214), dans lequel au moins l'une des chambres de dilution parmi la pluralité de chambres de dilution est en communication avec une région de chargement d'échantillon (205, 211) et le réservoir d'eau (206, 212).

10. Dispositif microfluidique selon la revendication 9, dans lequel la zone de préparation (201) comprend en outre une zone de rassemblement de réactifs de PCR (215) comprenant une pluralité de chambres de rassemblement (216), chacune en communication avec un ou plusieurs réservoirs de réactifs de PCR (217), dans lequel au moins une chambre de dilution de la pluralité de chambres de dilution (214) est en communication avec au moins une chambre de rassemblement de la pluralité de chambres de rassemblement.

11. Procédé d'exécution d'une PCR numérique sur un dispositif microfluidique (100, 200) basé sur l'électromouillage selon l'une quelconque des revendications 1 à 10, le procédé comprenant les étapes dans l'ordre suivant :
(a) ajout d'une partition comprenant un échantillon dans une zone de chargement d'échantillon (204, 210) positionnée sur le dispositif,
(b) ajout d'un volume d'eau à la partition dans la zone de chargement d'échantillon (204, 210) et donc dilution de la partition ;
(c) ajout d'un mélange de réactifs de PCR à la partition dans la zone de chargement d'échantillon (204, 210) ;
(d) partitionnement de la partition en une pluralité de partitions, dans lequel l'étape de partitionnement est effectuée dans une zone de génération de partitions (208) sur le dispositif ;
(e) soumission de la pluralité de partitions à un protocole thermique pour générer une ou plusieurs partitions contenant des amplicons, dans lequel l'étape de soumission (e) est effectuée dans une zone d'amplification (202, 220) sur le dispositif ; et
(f) soumission de la ou des partitions contenant des amplicons à un gradient thermique pour générer ainsi un profil de fusion pour chacune de la ou des partitions contenant des amplicons, dans lequel l'étape de soumission (f) est effectuée dans une zone de courbe de fusion (203, 221) sur le dispositif ;
(g) détermination d'une concentration d'un échantillon en se basant sur les résultats de l'étape (f) ; et
(h) répétition des étapes (a) à (g) avec une ou plusieurs partitions supplémentaires comprenant une taille de partition ajustée en fonction de la taille de partition précédente pour déterminer une concentration de l'échantillon ajustée jusqu'à l'obtention d'une distribution de Poisson optimale.

12. Procédé selon la revendication 11, dans lequel un premier ensemble de partitions est soumis aux étapes (a) à (g) et le ou plusieurs ensembles de partitions supplémentaires sont soumis aux étapes (a) à (g), dans lequel l'échantillon du ou des ensembles de partitions supplémentaires est dilué en série par rapport à l'échantillon du premier ensemble de partitions.

13. Procédé d'exécution d'une analyse par PCR numérique multiplexe sur un dispositif microfluidique (100, 200) basé sur l'électromouillage selon l'une quelconque des revendications 1 à 10, le procédé comprenant les étapes dans l'ordre suivant :
(a) ajout d'une partition comprenant un échantillon à une zone de chargement d'échantillon (204, 210) positionnée sur le dispositif, dans lequel l'échantillon comprend une pluralité de séquences cibles ;
(b) ajout d'un volume d'eau à la partition dans la zone de chargement d'échantillon (204, 210) et donc dilution de la partition ;
(c) ajout d'un mélange de réactifs de PCR à la partition dans la zone de chargement d'échantillon (204, 210) ;
(d) partitionnement de la partition en une pluralité de partitions, dans lequel l'étape de partitionnement est effectuée dans une zone de génération de partitions (208) sur le dispositif ;
(e) soumission de la pluralité de partitions à un protocole thermique pour générer une ou plusieurs partitions contenant des amplicons, dans lequel l'étape de soumission (e) est effectuée dans une zone d'amplification (202, 220) sur le dispositif ;
(f) soumission de la ou des partitions contenant des amplicons à un gradient thermique pour générer ainsi un profil de fusion pour chacune de la ou des partitions contenant des amplicons, dans lequel l'étape de soumission (f) est effectuée dans une zone de courbe de fusion (203, 221) sur le dispositif ;
(g) détection de la présence et/ou de l'absence de chacune des séquences cibles dans la pluralité de séquences cibles sur la base du profil de fusion de chacun du ou des amplicons ; et
(h) répétition des étapes (a) à (g) avec une ou plusieurs partitions supplémentaires comprenant une taille de partition ajustée en fonction de la taille de partition précédente jusqu'à l'obtention d'une distribution de Poisson optimale.

14. Procédé selon la revendication 13, dans lequel un premier ensemble de partitions est soumis aux étapes (a) à (g) et le ou les ensembles de partitions supplémentaires sont soumis aux étapes (a) à (g), dans lequel l'échantillon du ou des ensembles de partitions supplémentaires est dilué en série par rapport à l'échantillon du premier ensemble de partitions.

15. Procédé selon l'une quelconque des revendications 11 à 14, dans lequel après l'étape (e), chacune de la pluralité de partitions comprend zéro ou une séquence cible.
